# EUROPEAN PATENT SPECIFICATION

(11) **EP 1 960 427 B1**
(45) Date of publication and mention of the grant of the patent: **23.02.2011**
(21) Application number: 06828004.9
(22) Date of filing: 13.12.2006
(51) Int. Cl.: C07K 14/735, C07K 14/47, C07K 19/00, A61P 37/06, C07K 17/00, C12N 15/62

(54) **MULTIMERIC FC RECEPTOR POLYPEPTIDES**
MULTIMERE FC-REZEPTOR-POLYPEPTIDE
POLYPEPTIDES POLYMERES POUR RECEPTEURS FC

(30) Priority: 13.12.2005 US 750301 P
(43) Date of publication of application: 27.08.2008
(73) Proprietor: SuppreMol GmbH, 82152 Martinsried (DE)
(72) Inventor: HOGARTH, Phillip, Mark, Williamstown, Victoria 3016 (AU); WINES, Bruce, David, Heidelberg, Victoria 3084 (AU)
(74) Representative: Kilger, Christian
(86) International application number: PCT/AU2006/001890
(87) International publication number: WO 2007/068047

(56) References cited:
- WO-A1-91/06570
- WO-A2-2004/062619
- KURUCZ ISTVAN ET AL: "Bacterially expressed human FcgammaRIIb is soluble and functionally active after in vitro refolding" IMMUNOLOGY LETTERS, vol. 75, no. 1, 1 December 2000 (2000-12-01), pages 33-40, XP002511480 ISSN: 0165-2478
- IERINO F L ET AL: "RECOMBINANT SOLUBLE HUMAN FCGAMMARII: PRODUCTION, CHARACTERIZATION AND INHIBITION OF THE ARTHUS REACTION" JOURNAL OF EXPERIMENTAL MEDICINE, ROCKEFELLER UNIVERSITY PRESS, JP, vol. 178, no. 5, 1 November 1993 (1993-11-01), pages 1617-1628, XP009015491 ISSN: 0022-1007
- POWELL M S ET AL: "Investigation of FcgammaRIIa dimerization" FASEB JOURNAL, vol. 14, no. 6, 20 April 2000 (2000-04-20), page A960, XP009111142 & JOINT ANNUAL MEETING OF THE AMERICAN ASSOCIATION OF IMMUNOLOGISTS AND THE CLINICAL IMMUNOLOGY SOCIET; SEATTLE, WASHINGTON, USA; MAY 12-16, 2000 ISSN: 0892-6638
- LI P. ET AL.: 'Recombinant CD16A-Ig forms a homodimer and cross-blocks the ligand binding functions of neutrophil and monocyte Fcgamma receptors' MOLECULAR IMMUNOLOGY vol. 38, 2001, pages 527 - 538, XP002995781
- KERSHAW M.H. ET AL.: 'Redirected Cytotoxic Effector Function' THE JOURNAL OF BIOLOGICAL CHEMISTRY vol. 271, no. 35, 1996, pages 21214 - 21220, XP003014118
- HARRISON P.T. AND ALLEN J.M.: 'High affinity IgG binding by FcgammaRI (CD64) is modulated by two distinct IgSF domains and the transmembrane domain of the receptor' PROTEIN ENGINEERING vol. 11, no. 3, 1998, pages 225 - 232, XP003014119
- HULETT M.D. ET AL.: 'Multiple Regions of Human FcgammaRII (CD32) Contribute to the Binding of IgG' THE JOURNAL OF BIOLOGICAL CHEMISTRY vol. 270, no. 36, 1995, pages 21188 - 21194, XP002139929
- MAXWELL K.F. ET AL.: 'Crystal structure of the human leukocyte Fc receptor, FcgammaRIIa' NATURE STRUCTURAL BIOLOGY vol. 6, no. 5, 1999, pages 437 - 442, XP003005007
- POWELL M.S. ET AL.: 'Alteration of the FcgammaRIIa Dimer Interface Affects Receptor Signaling but Not Ligand Binding' THE JOURNAL OF IMMUNOLOGY vol. 176, no. 12, 2006, pages 7489 - 7494, XP003014120

### SEQUENCE LISTING

<110> Trillium Therapeutics Inc
<120> MULTIMERIC FC RECEPTOR POLYPEPTIDES
<130> 30109PCT
<160> 6
<170> PatentIn version 3.3
<210> 1
   <211> 19
   <212> DNA
   <213> Homo sapiens
<400> 1
   gtagctcccc caaaggctg 19
<210> 2
   <211> 25
   <212> DNA
   <213> Homo sapiens
<400> 2
   ctacccgggt gaagagctgc ccatg 25
<210> 3
   <211> 48
   <212> DNA
   <213> Homo sapiens
<400> 3
   tctcatcacc accatcacca cgtctagacc cagctttctt gtacaaag 48
<210> 4
   <211> 10
   <212> PRT
   <213> Artificial
<220>
   <223> synthetic linker
<400> 4
   Gly Gly Gly Gly Ser Gly Gly Gly Gly Ser
   1               5                   10
<210> 5
   <211> 1206
   <212> DNA
   <213> Homo sapiens
<400> 5
   atggagaccc aaatgtctca gaatgtatgt cccagaaacc tgtggctgct tcaaccattg     60
   acagttttgc tgctgctggc ttctgcagac agtcaagctg cagctccccc aaaggctgtg    120
   ctgaaacttg agcccccgtg gatcaacgtg ctccaggagg actctgtgac tctgacatgc    180
   cagggggctc gcagccctga gagcgactcc attcagtggt tccacaatgg gaatctcatt     240
   cccacccaca cgcagcccag ctacaggttc aaggccaaca acaatgacag cggggagtac    300
   acgtgccaga ctggccagac cagcctcagc gaccctgtgc atctgactgt gctttccgaa    360
   tggctggtgc tccagacccc tcacctggag ttccaggagg gagaaaccat catgctgagg    420
   tgccacagct ggaaggacaa gcctctggtc aaggtcacat tcttccagaa tggaaaatcc    480
   cagaaattct cccatttgga tcccaccttc tccatcccac aagcaaacca cagtcacagt    540
   ggtgattacc actgcacagg aaacataggc tacacgctgt tctcatccaa gcctgtgacc    600
   atcactgtcc aagtgcccag catgggcagc tcttcacccg tagctccccc aaaggctgtg    660
   ctgaaacttg agcccccgtg gatcaacgtg ctccaggagg actctgtgac tctgacatgc    720
   cagggggctc gcagccctga gagcgactcc attcagtggt tccacaatgg gaatctcatt     780
   cccacccaca cgcagcccag ctacaggttc aaggccaaca acaatgacag cggggagtac    840
   acgtgccaga ctggccagac cagcctcagc gaccctgtgc atctgactgt gctttccgaa    900
   tggctggtgc tccagacccc tcacctggag ttccaggagg gagaaaccat catgctgagg    960
   tgccacagct ggaaggacaa gcctctggtc aaggtcacat tcttccagaa tggaaaatcc   1020
   cagaaattct cccatttgga tcccaccttc tccatcccac aagcaaacca cagtcacagt   1080
   ggtgattacc actgcacagg aaacataggc tacacgctgt tctcatccaa gcctgtgacc   1140
   atcactgtcc aagtgcccag catgggcagc tcttcaccct ctcatcacca ccatcaccac   1200
   gtctag                                                              1206
<210> 6
   <211> 401
   <212> PRT
   <213> Homo sapiens
<400> 6
   Met Glu Thr Gln Met Ser Gln Asn Val Cys Pro Arg Asn Leu Trp Leu
   1               5                   10                  15
   Leu Gln Pro Leu Thr Val Leu Leu Leu Leu Ala Ser Ala Asp Ser Gln
               20                  25                  30
   Ala Ala Ala Pro Pro Lys Ala Val Leu Lys Leu Glu Pro Pro Trp Ile
           35                  40                  45
   Asn Val Leu Gln Glu Asp Ser Val Thr Leu Thr Cys Gln Gly Ala Arg
       50                  55                  60
   Ser Pro Glu Ser Asp Ser Ile Gln Trp Phe His Asn Gly Asn Leu Ile
   65                  70                  75                  80
   Pro Thr His Thr Gln Pro Ser Tyr Arg Phe Lys Ala Asn Asn Asn Asp
                   85                  90                  95
   Ser Gly Glu Tyr Thr Cys Gln Thr Gly Gln Thr Ser Leu Ser Asp Pro
               100                 105                 110
   Val His Leu Thr Val Leu Ser Glu Trp Leu Val Leu Gln Thr Pro His
           115                 120                 125
   Leu Glu Phe Gln Glu Gly Glu Thr Ile Met Leu Arg Cys His Ser Trp
       130                 135                 140
   Lys Asp Lys Pro Leu Val Lys Val Thr Phe Phe Gln Asn Gly Lys Ser
   145                 150                 155                 160
   Gln Lys Phe Ser His Leu Asp Pro Thr Phe Ser Ile Pro Gln Ala Asn
                   165                 170                 175
   His Ser His Ser Gly Asp Tyr His Cys Thr Gly Asn Ile Gly Tyr Thr
               180                 185                 190
   Leu Phe Ser Ser Lys Pro Val Thr Ile Thr Val Gln Val Pro Ser Met
           195                 200                 205
   Gly Ser Ser Ser Pro Val Ala Pro Pro Lys Ala Val Leu Lys Leu Glu
       210                 215                 220
   Pro Pro Trp Ile Asn Val Leu Gln Glu Asp Ser Val Thr Leu Thr Cys
   225                 230                 235                 240
   Gln Gly Ala Arg Ser Pro Glu Ser Asp Ser Ile Gln Trp Phe His Asn
                   245                 250                 255
   Gly Asn Leu Ile Pro Thr His Thr Gln Pro Ser Tyr Arg Phe Lys Ala
               260                 265                 270
   Asn Asn Asn Asp Ser Gly Glu Tyr Thr Cys Gln Thr Gly Gln Thr Ser
           275                 280                 285
   Leu Ser Asp Pro Val His Leu Thr Val Leu Ser Glu Trp Leu Val Leu
       290                 295                 300
   Gln Thr Pro His Leu Glu Phe Gln Glu Gly Glu Thr Ile Met Leu Arg
   305                 310                 315                 320
   Cys His Ser Trp Lys Asp Lys Pro Leu Val Lys Val Thr Phe Phe Gln
                   325                 330                 335
   Asn Gly Lys Ser Gln Lys Phe Ser His Leu Asp Pro Thr Phe Ser Ile
               340                 345                 350
   Pro Gln Ala Asn His Ser His Ser Gly Asp Tyr His Cys Thr Gly Asn
           355                 360                 365
   Ile Gly Tyr Thr Leu Phe Ser Ser Lys Pro Val Thr Ile Thr Val Gln
       370                 375                 380
   Val Pro Ser Met Gly Ser Ser Ser Pro Ser His His His His His His
   385                 390                 395                 400
   Val

## Description

### FIELD OF THE INVENTION

The present invention relates to soluble multimeric Fc receptor polypeptides able to inhibit leukocyte Fcγ receptors (FcγR) and immunoglobulin G (IgG) interactions. Such polypeptides are useful in the treatment of inflammatory diseases, particularly immune complex-mediated inflammatory diseases such as rheumatoid arthritis (RA), immune thrombocytopenic purpura (ITP) and systemic lupus erythematosus (SLE).

### BACKGROUND OF THE INVENTION

The treatment of autoimmune and other inflammatory diseases such as RA and SLE has entered a new and exciting phase where increased understanding of the molecules involved in the immune system has allowed for the specific inhibition of key inflammatory molecules such as tumour necrosis factor-α (TNFα) and interleukin 1β (IL-1β). For example, in recent studies, it has been shown that antibodies can play a powerful role in the pathogenesis of RA, and in human clinical trials, positive responses to the use of anti-CD20 monoclonal antibody (MAb) therapy to eliminate antibody producing B cells have been generating strong evidence of the significant role of antibodies in RA (Emery *et al.,* 2001). Since Fc receptors (FcR) play pivotal roles in immunoglobulin-based effector systems, inhibition of FcR function may provide the basis of effective therapy for a variety of diseases. Moreover, since Fcγ receptors (FcγR) are pivotal to effector systems for IgG, targeting the interaction between leukocyte FcγRs and antibodies provides a new opportunity for therapeutic intervention in RA (Nabbe *et al.,* 2003). One approach of achieving such an intervention which is of interest to the present applicant is the use of a soluble form of an FcγR to act as a "decoy" to prevent leukocyte activation by antibodies.

Fc receptors (FcR) are leukocyte surface glycoproteins that specifically bind the Fc portion of antibodies. The receptors for IgG, that is FcγR, are the most widespread and diverse, the major types being FcγRI (CD64), FcγRII (CD32) and FcγRIII (CD16). Immune complexes (IC) that are formed *in vivo* in normal immune responses, and those seen in the pathology of autoimmune diseases such as RA, can simultaneously engage many FcR. For example, in humans, activated macrophages, neutrophils, eosinophils and mast cells can express PcγRI, FcγRIIa, FcγRIIb and FcγRIII (Takai, 2002). However, of these, the FcγRIIa is the major initiator of IC-mediated inflammation and, while all of the FcγR types engage the lower hinge region of the IgG Fc domain and the CH2 domains such that any soluble FcγR decoy polypeptide might inhibit the binding of IgG to all classes of FcγR, the present applicant has realised that since FcγRIIa shows the widest binding specificity and highest selectivity for avid IgG immune complex binding, the development and investigation of a soluble FcγRIIa offers the greatest potential.

WO-A2 2004/062619 and Li P et al. (2001) disclose homodimeric fusion proteins of Fc binding regions each fused to hinge domain of IgG₂. The dimerisation of these fusion proteins occurs via disulfide bridges between the hinge domains.

Indeed, previous studies have shown that a simple recombinant soluble FcγRIIa polypeptide (rsFcγRIIa monomer), consisting of FcγRIIa ectodomains (Ierino *et al.,* 1993a), is clearly able to inhibit IC-mediated inflammation. In these studies, the rsFcγRIIa was tested using the Arthus reaction, wherein immune complexes are formed in the dermis by the passive administration of antibody and antigen (Pflum *et al.,* 1979), which is a model of vasculitis (an extra articular complication in arthritis) and also occurs in SLE. It was found that while the rsFcγRIIa monomer inhibited inflammation and neutrophil infiltration when co-administered with the antibody and antigen, large amounts of the rsFcγRIIa monomer were required because of a relatively low level of selectivity for the immune complexes. To overcome this problem, the present applicant proposed to use a multimeric form of the rsFcγRIIa decoy, and has since found, surprisingly, that not only could such a multimeric form be successfully expressed, it exhibits increased selectivity for immune complexes. Such multimeric rsFcγRIIa polypeptides therefore show considerable promise for the treatment of IC-mediated inflammatory disease such as RA and SLE.

### SUMMARY OF THE INVENTION

Thus, in a first aspect, the present invention provides a soluble multimeric protein or polypeptide able to inhibit interaction of leukocyte Fcγ receptors (FcγR) and immunoglobulin G (IgG), said protein or polypeptide comprising two linked Fc binding regions, at least one of which is derived from an FcγR type receptor.

Preferably, the protein or polypeptide is a multimer of an Fc binding region derived from an FcγRII type receptor, particularly FcγRIIa. Such a molecule may be considered to be a homomultimer, and one especially preferred molecule of this kind is a homodimer of an Fc binding region derived from an FcγRII type receptor. However, the present invention also contemplates that the molecule may be a multimer of an Fc binding region derived from an FcγR type receptor (e.g. an FcγRII type receptor) and an Fc binding region from another source (e.g. an Fc binding region from another Fc receptor type or a synthetic Fc binding polypeptide). A molecule of this kind may be considered to be a heteromultimer, and one especially preferred molecule of this kind is a heterodimer of an Fc binding region derived from an FcγRII type receptor and an Fc binding region derived from an FcγRIII type receptor.

The Fc binding regions are linked through a peptide bond or via a short linker sequence (e.g. a single amino acid or a short peptide of, for example, 2 to 20 amino acids in length).

In a second aspect, the present invention provides a polynucleotide molecule comprising a nucleotide sequence encoding a soluble multimeric protein or polypeptide according to the first aspect.

The polynucleotide molecule may consist in an expression cassette or expression vector (e.g. a plasmid for introduction into a bacterial host cell, or a viral vector such as a baculovirus vector for transfection of an insect host cell, or a plasmid or viral vector such as a lentivirus for transfection of a mammalian host cell).

Thus, in a third aspect, the present invention provides a recombinant host cell containing a polynucleotide molecule comprising a nucleotide sequence encoding a soluble multimeric protein or polypeptide according to the first aspect.

In a fourth aspect, the present invention provides a method for producing a protein or polypeptide, the method comprising the steps of;
(i) providing a recombinant host cell containing a polynucleotide molecule comprising a nucleotide sequence encoding a soluble multimeric protein or polypeptide according to the first aspect,
(ii) culturing said host cell in a suitable culture medium and under conditions suitable for expression of said soluble multimeric protein or polypeptide, and
(iii) isolating said soluble multimeric protein or polypeptide from the culture medium.

In a fifth aspect, the present invention provides a method of treating a subject for an inflammatory disease, said method comprising administering to said subject a soluble multimeric protein or polypeptide according to the first aspect optionally in combination with a pharmaceutically- or veterinary-acceptable carrier or excipient.

### BRIEF DESCRIPTION OF THE FIGURES

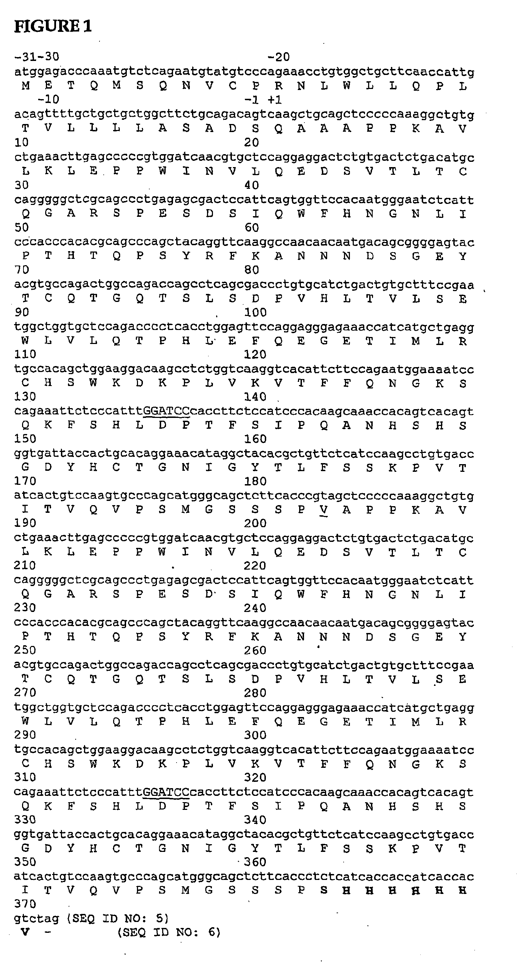

**Figure 1** provides the nucleotide sequence (and translated amino acid sequence) for a head to tail homodimer construct of two FcγRIIa extracellular regions each comprising both FcγRIIa ectodomains, namely ectodomains 1 and 2. The FcγRIIa ectodomains 1 and 2 consist of amino acids 1 to 174 of the FcγRIIa polypeptide sequence with amino acids 1 to 88 comprising domain 1 and amino acids 89 to 174 comprising domain 2 (Hibbs *et al.,* 1988; *Homo sapiens* Fc fragment of IgG, low affinity IIa receptor (CD32) (FCGR2A), mRNA, ACCESSION NM_021642; and Powell *et al.*, 1999). In the figure, amino acids 1 to 182 are derived from the extracellular region of FcγRIIa, of which amino acids 1 to 174 comprise the FcγRIIa ectodomains 1 and 2 and amino acids 175 to 182 comprise the membrane proximal stalk (which in FcγRIIa links the ectodomains 1 and 2 to the transmembrane sequence). The first of the FcγRIIa extracellular regions comprising the dimer therefore consists of amino acids 1 to 182 and the second of the FcγRIIa extracellular regions consists of amino acids 184 to 362 (corresponding to amino acids 3 to 182 of FcγRIIa). The underlined amino acid represents a non-FcγRIIa linker amino acid residue, while the bolded amino acids highlight a C-terminal His₆ tag.

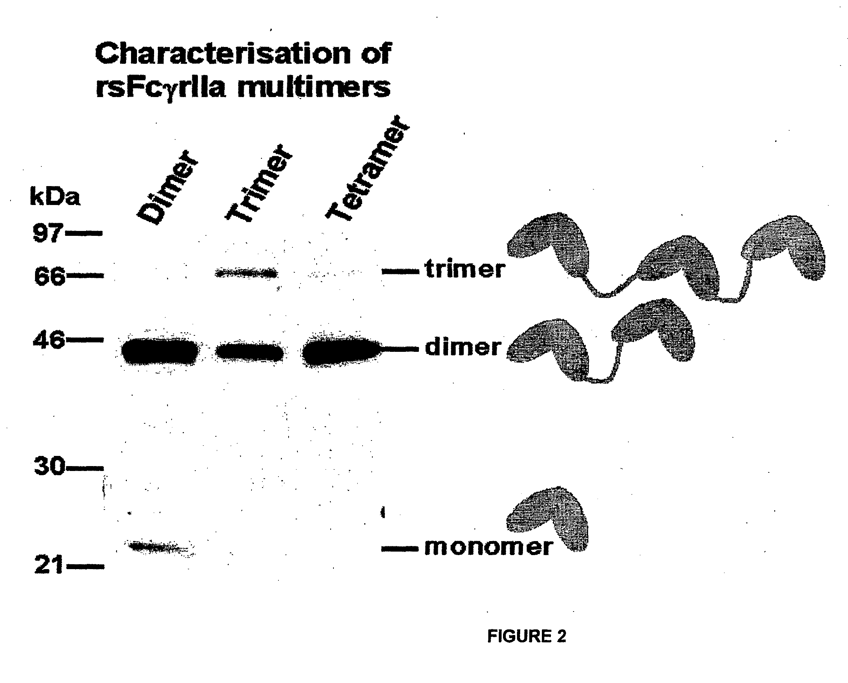

**Figure 2** shows a Western Blot analysis of recombinant soluble (rs) multimeric forms of FcγRIIa. The rsFcγRIIa dimer was substantially stable with only a small amount of rsFcγRIIa monomer breakdown product evident. On the other hand, the rsFcγRIIa trimer and tetramer forms were unstable, being substantially degraded to the rsFcγRIIa dimer form. This degradation may be avoided by the use of protease inhibitors during production or by otherwise modifying the sequence of the multimer forms so as to remove cleavage site(s).

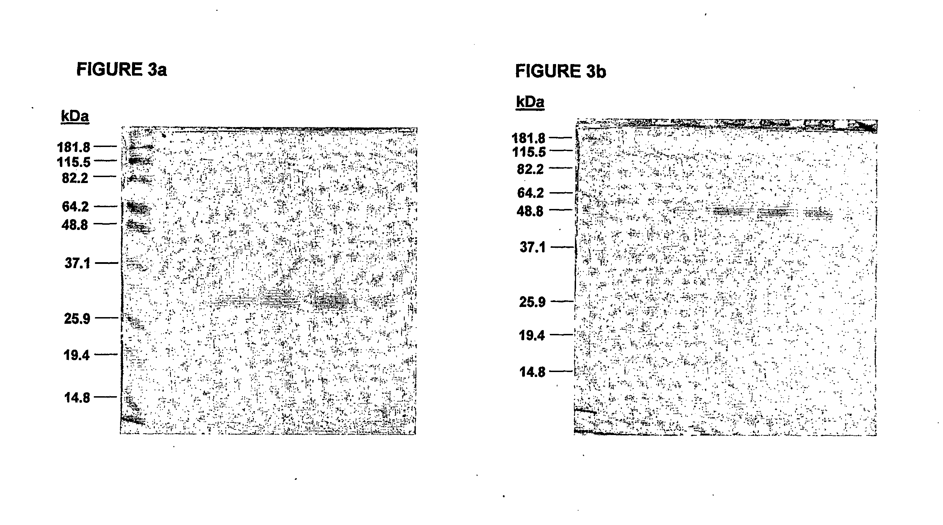

**Figure 3** shows a Coomassie-stained SDS-PAGE (12% acrylamide gel, under non-reducing conditions) of fractions collected from the purification of rsFcγRIIa monomer (expressed from mammalian cells) having the expected size of -30 kDa (a), and rsFcγRIIa dimer having the expected size of -50 kDa (b).

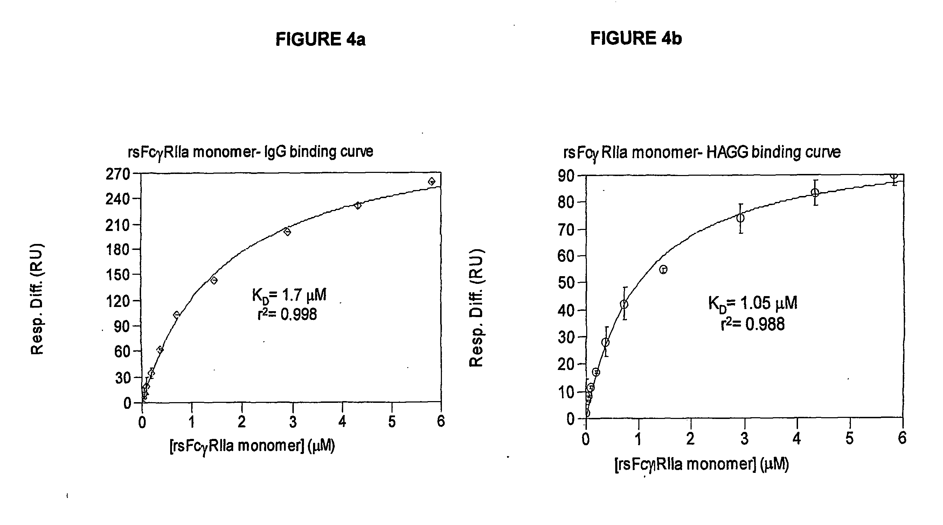

**Figure 4** graphically shows the equilibrium binding responses of rsFcγRIIa monomer to immobilised (a) IgG monomer (Sandoglobulin) and (b) the model immune complex, heat-aggregated IgG (HAGG).

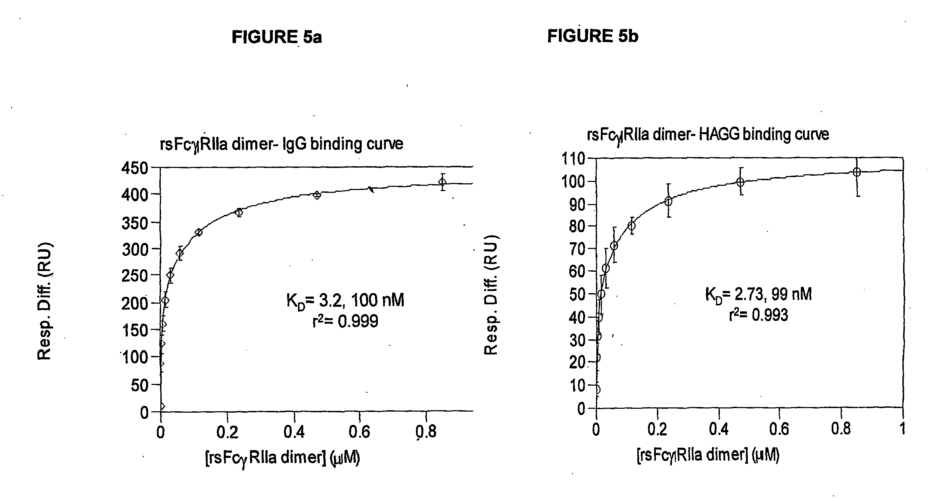

**Figure 5** graphically shows the equilibrium binding responses of rsFcγRIIa dimer to immobilised (a) IgG monomer (Sandoglobulin) and (b) the model immune complex, HAGG.

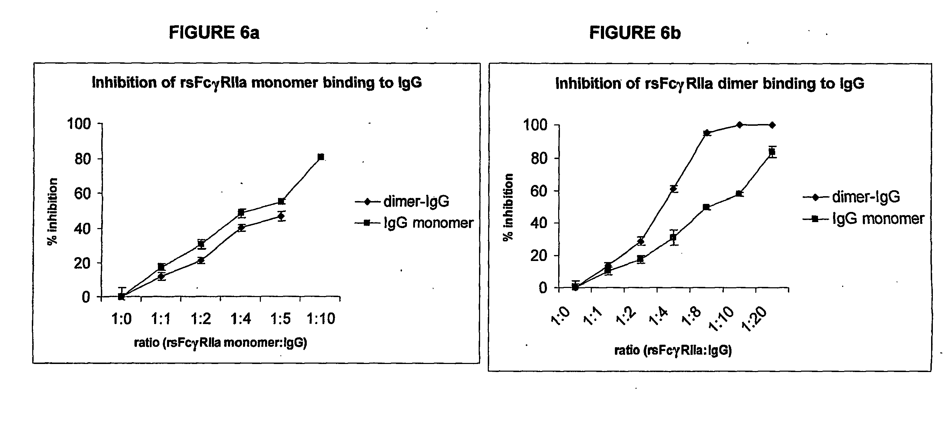

**Figure 6** provides a plot of rsFcγRIIa monomer (a) and rsFcγRIIa dimer (b) binding to immobilised human IgG monomer (Sandoglobulin) following prior reaction in solution with human IgG monomer (Sandoglobulin) and dimer-IgG (Wright *et al.*, 1980), as determined using a standard BIAcore assay protocol.

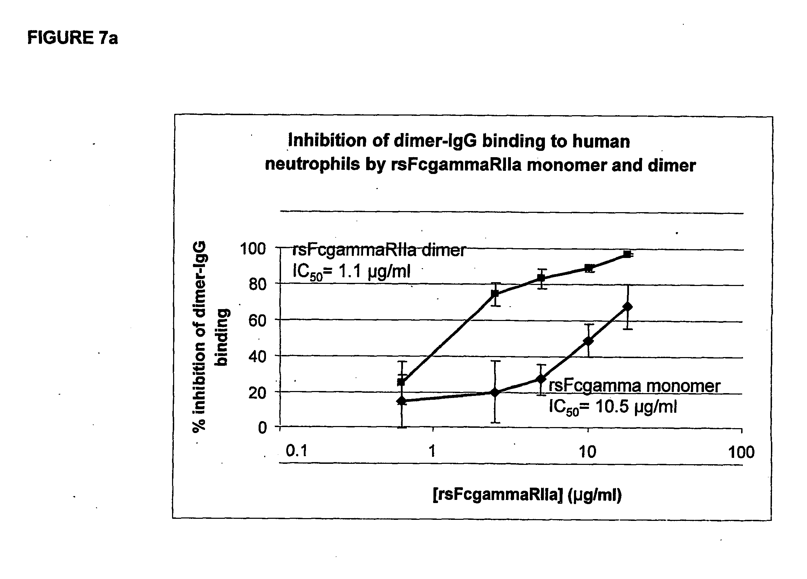

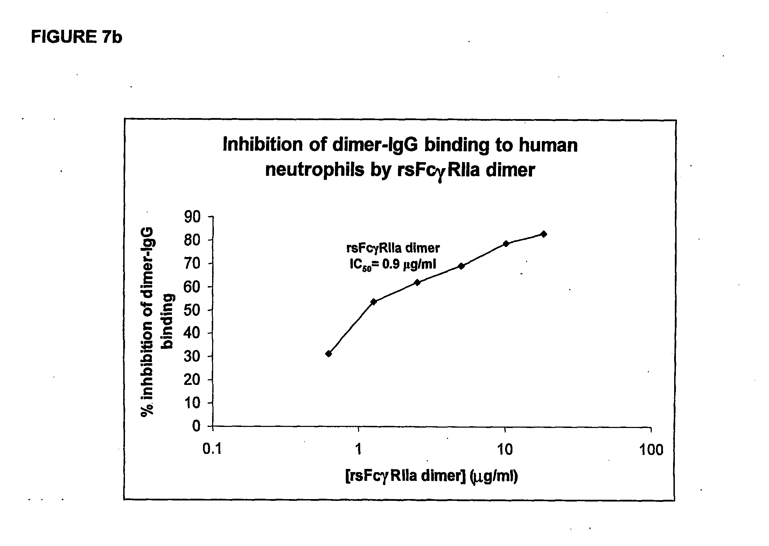

**Figure 7** provides plots of (a) the inhibition of dimer-IgG (Wright *et al.*, 1980) binding to human neutrophils (volunteer V5) by purified rsFcγRIIa monomer and rsFcγRIIa dimer calculated as a percentage of the uninhibited dimer-IgG binding activity and (b) the inhibition of dimer-IgG binding to human neutrophils (volunteer V1) by purified rsFcγRIIa dimer calculated as a percentage of dimer-IgG binding dimer.

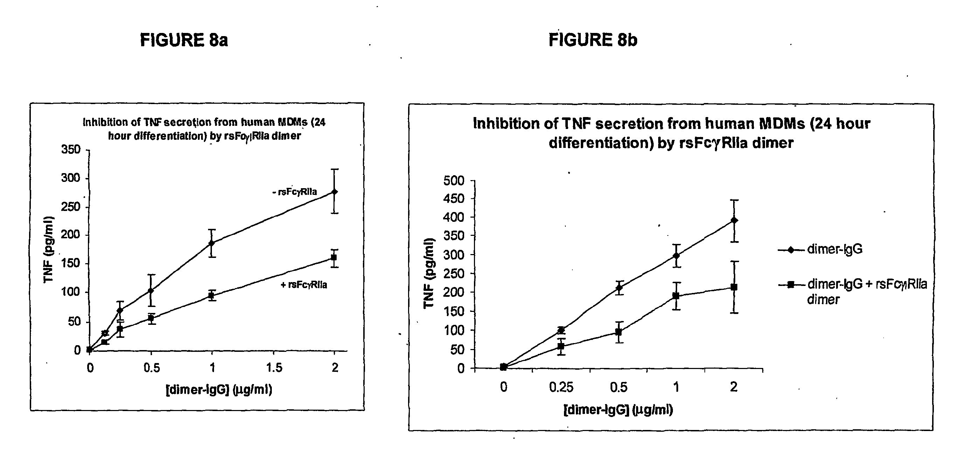

**Figure 8** provides at (a), a plot of immune-complex (dimer-IgG) stimulated TNF secretion from 24 hour differentiated human MDMs (volunteer V5) in the absence and presence of rsFcγRIIa dimer (in supernatant at 2.5 µg/ml); while at (b), provides a plot of immune-complex (dimer-IgG) stimulated TNF secretion from 24 hour differentiated human MDMs (volunteer V1), in the absence and presence of rsFcγRIIa dimer (2.5 µg/ml).

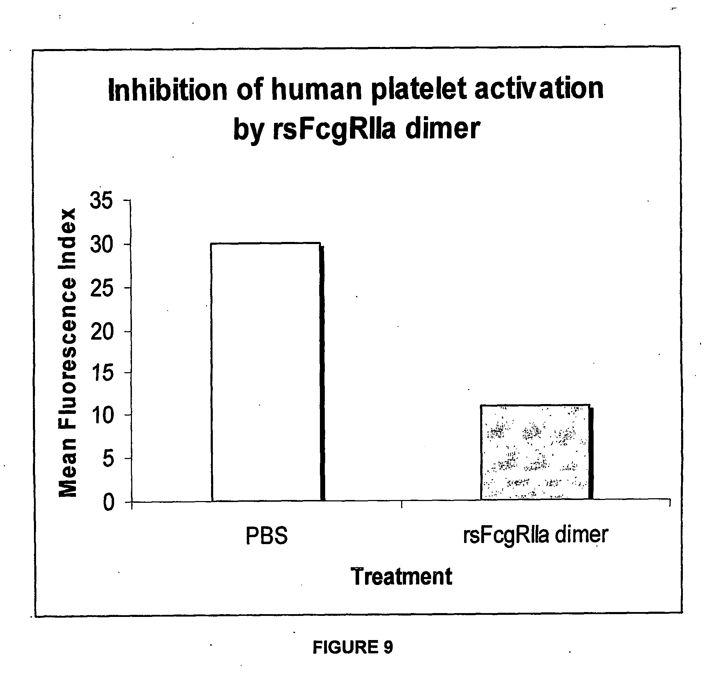

**Figure 9** provides a plot of immune-complex (HAGG) stimulated activation of human platelets, as measured by the mean fluorescence intensity (MFI) of P-selectin expression in the absence and presence of rsFcγRIIa dimer (30 µg/ml).

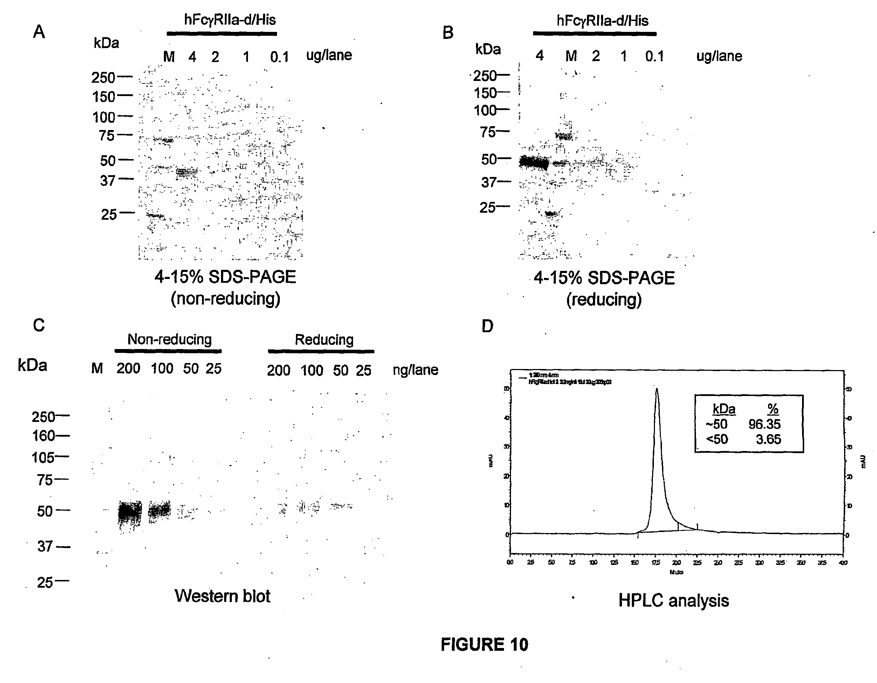

**Figure 10** provides results from the analysis of the rsFcγRIIa dimer isolated from stably transfected CHO-S cells by SDS-PAGE under (a) non-reducing and (b) reducing conditions, (c) Western blotting using an anti-FcγRIIa antibody, and (d) HPLC. The rsFcγRIIa dimer migrates as a single band at the expected molecular weight (~ 50 kD), reacts with anti-FcγRIIa antibody and was >96% pure as determined by HPLC analysis.

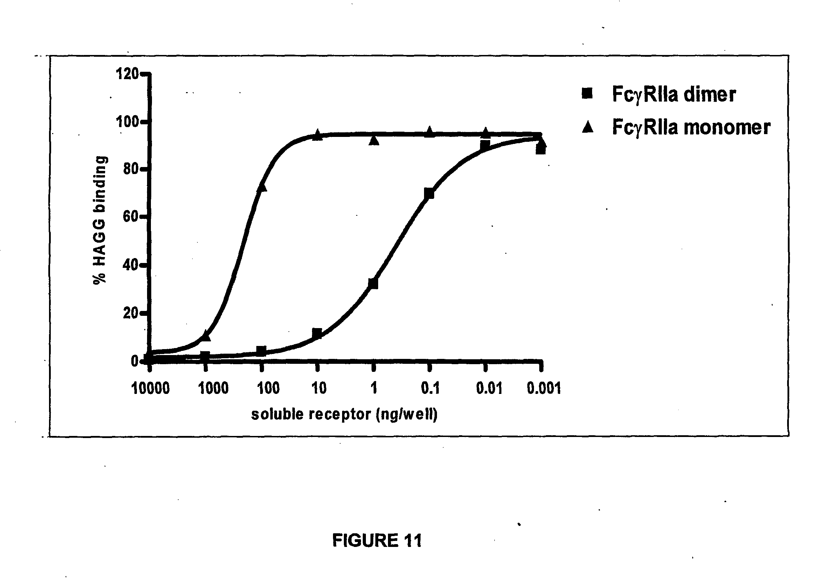

**Figure 11** provides a plot of immune-complex (HAGG) binding to cell surface-expressed human FcγRIIb (on the murine B lymphoma cell line IIA1.6) in the presence of either rsFcγRIIa monomer or rsFcγRIIa dimer.

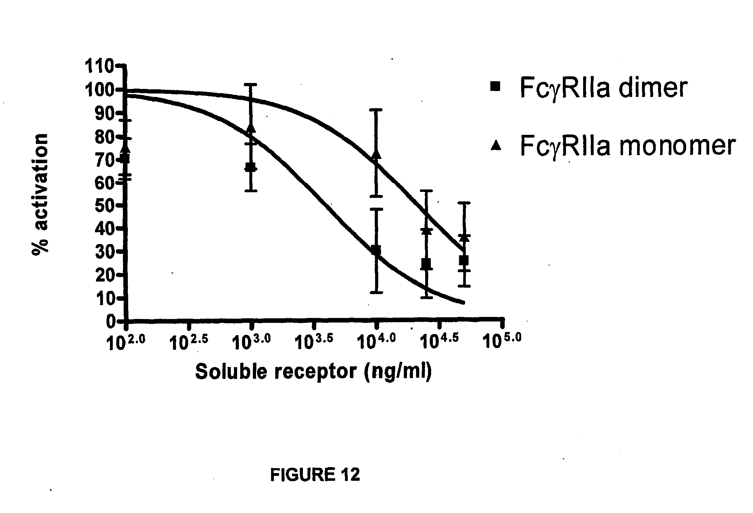

**Figure 12** provides a plot of activated platelets (positive for both CD41 and CD62P) after treatment with HAGG in the presence of rsFcγRIIa monomer or rsFcγRIIa dimer, as a percentage of activated platelets following treatment with HAGG alone.

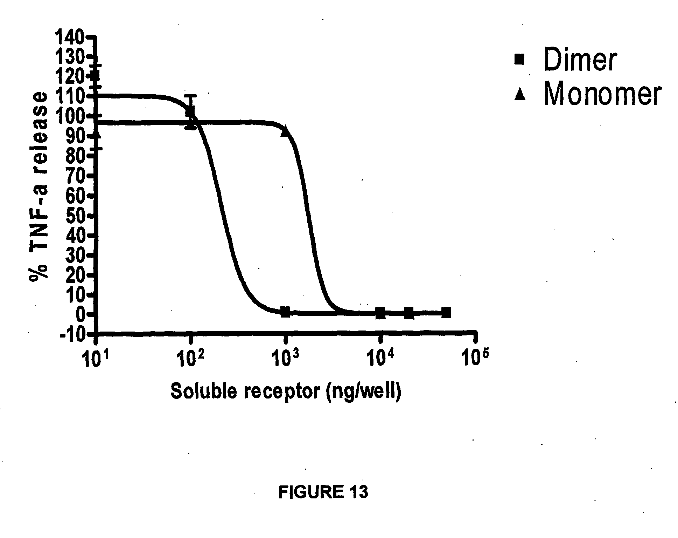

**Figure 13** provides a plot of TNF-α release from MC/9 cells after incubation with OVA immune complexes in the presence of rsFcγRIIa monomer or rsFcγRIIa dimer, as a percentage of TNF-α released in the presence of OVA immune complexes alone.

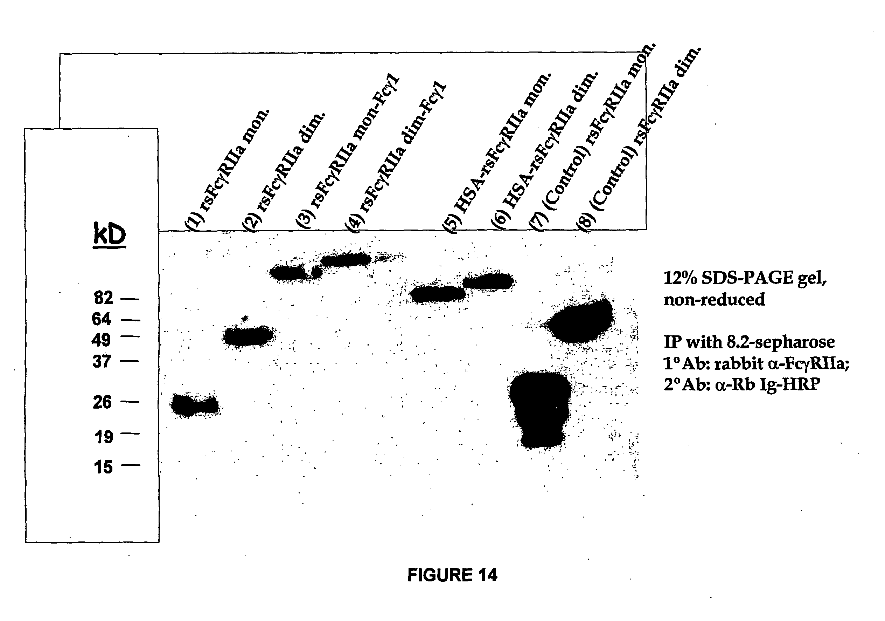

**Figure 14** shows Western blot analysis of rsFcγRIIa fusion proteins. (1) rsFcγRIIa monomer; (2) rsFcγRIIa dimer; (3) rsFcγRIIa monomer fused to IgG₁-Pcγ1 (L234A, L235A); (4) rsFcγRIIa dimer fused to IgG₁-Fcγ1 (L234A, L235A); (5) rsFcγRIIa monomer fused to human serum albumin (HSA); (6) rsFcγyRIIa dimer fused to HSA; (7) purified rsFcγRIIa monomer standard; and (8) purified rsFcγRIIa dimer standard.

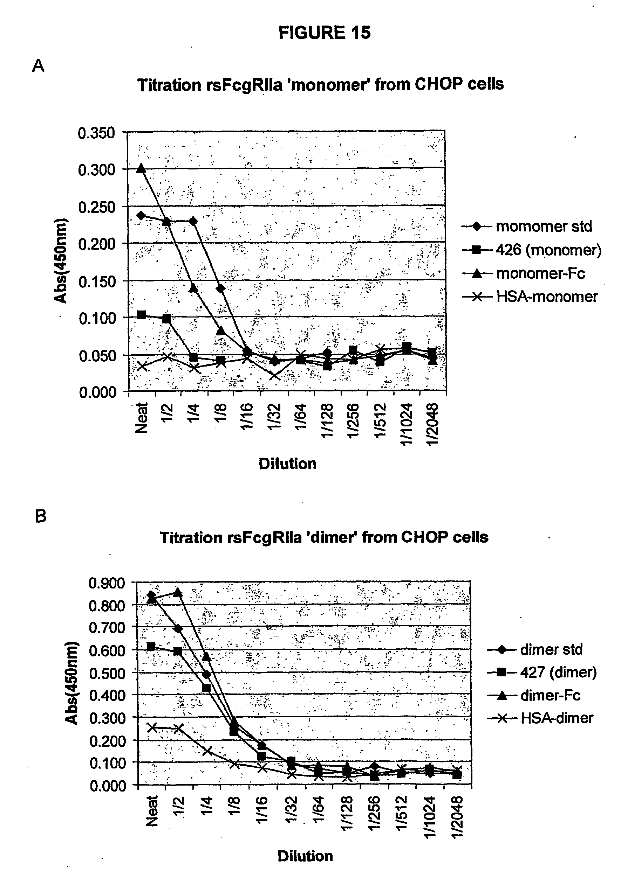

**Figure 15** provides the results of a HAGG-capture ELISA with rsFcγRIIa monomer and rsFcγRIIa dimer fusions. (a) FcγRIIa monomer standard (Powell *et al.,* 1999) starting at 0.75 µg/ml (monomer std); protein from cells transfected with rsFcγRIIa monomer construct (transfection 426 (monomer)); protein from cells transfected with rsFcγRIIa monomer fusion to IgG-Fcγ1 (L234A, L235A) construct (monomer-Fc); and protein from cells transfected with rsFcγRIIa monomer fusion to HSA construct (HSA-monomer); (b) rsFcγRIIa dimer standard starting at 0.5 µg/ml (dimer std); supernatant from cells transfected with rsFcγRIIa dimer (transfection 427 (dimer)); supernatant from cells transfected with rsFcγRIIa dimer fusion to IgG-Fcγ1 (L234A, L235A) (dimer-Fc); and supernatant from cells transfected with rsFcγRIIa dimer fusion to HSA (HSA-dimer).

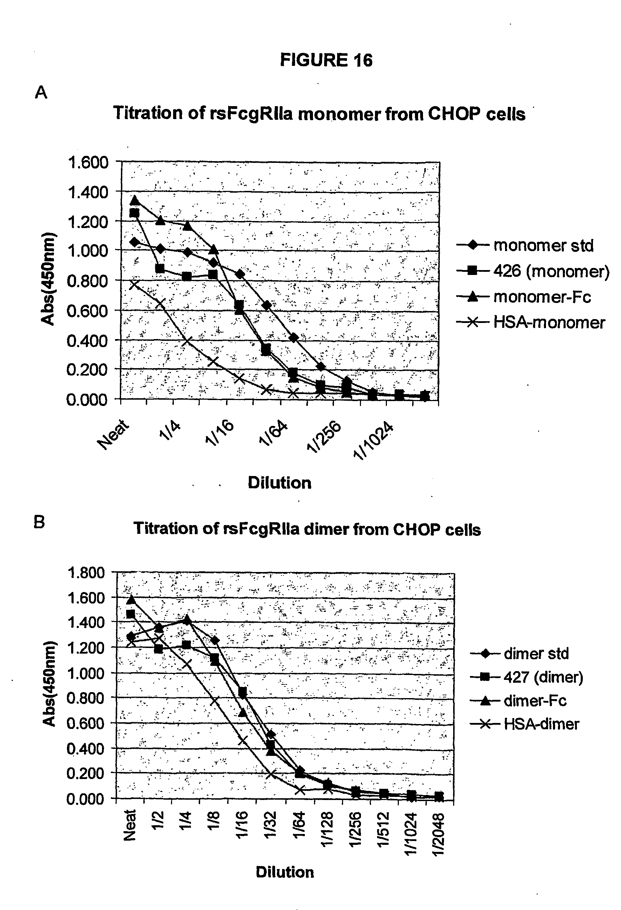

**Figure 16** provides results obtained from a CAPTURE-TAG ELISA on rsFcγRIIa monomer and rsFcγRIIa dimer fusion proteins to confirm the presence of epitopes that establish that the receptor is properly folded. (A) rsFcγRIIa monomer standard starting at 0.75 µg/ml (monomer std); supernatant from cells transfected with rsFcγRIIa monomer (transfection 426 (monomer)); supernatant from cells transfected with rsFcyRIIa monomer fusion to IgG-Fcγ1 (L234A, L235A) (monomer-Fc); and supernatant from cells transfected with rsFcγRIIa monomer fusion to HSA (HSA-monomer); (B) rsFcγRIIa dimer standard (prepared in-house) starting at 0.5 µg/ml); supernatant from cells transfected with PcγRIIa dimer (transfection 427 (dimer)); supernatant from cells transfected with rsFcγRIIa dimer fusion to IgG-Fcγ1 (L234A, L235A) (dimer-Fc); and supernatant from cells transfected with rsFcγRIIa dimer fusion to HSA (HSA-dimer).

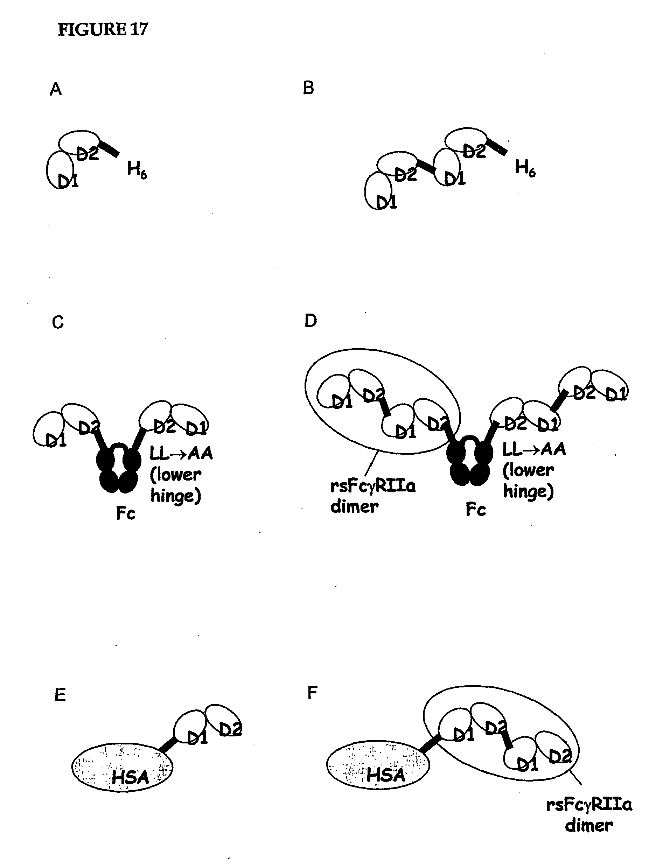

**Figure 17** provides a schematic diagram of (a) rsFcγRIIa monomer; (b) rsFcγRIIa dimer; (c) a dimer of a rsFcγRIIa monomer fusion to IgG-Fcγ1 (L234A, L235A), wherein the dimerisation is effected through the Fc domains of the rsFcγRIIa monomer fusion polypeptides, providing a molecule having two Fc binding regions (i.e. a protein that is dimeric for the Fc binding region or, otherwise, has a "valency of two"); (d) a dimer of rsFcγRIIa dimer fusion to IgG-Fcγ1 (L234A, L235A), wherein dimerisation is effected through the two Fc domains of the rsFcγRIIa dimer fusion polypeptides, providing a molecule having four Fc binding regions (i.e. a protein that is tetrameric for the Fc binding region or, otherwise, has a "valence of four"); (e) rsFcγRIIa monomer fusion to HSA; and (f) rsFcγRIIa dimer fusion to HSA. In the figure, D1 and D2 refers to, respectively, ectodomains 1 and 2, the solid bar shown adjacent to D2 represents a linker sequence, the dark loop at the top of the dimerised Fc domains in (c) and (d) represents disulphide linkages, and H₆ refers to a His tag).

### DETAILED DESCRIPTION OF THE INVENTION

The present invention provides a soluble multimeric protein or polypeptide able to inhibit interaction of leukocyte Fcγ receptors (FcγR) and immunoglobulin G (IgG) which comprises two Fc binding regions, one of which is essentially derived from an FcγR type receptor. Such a protein or polypeptide offers an increase in selectivity for immune complexes over that previously observed with soluble monomeric polypeptides such as rsFcγRII monomer, and thereby provides considerable promise as a "decoy" molecule for the treatment of IC-mediated inflammatory disease such as RA and SLE.

In a first aspect, the present invention therefore provides a soluble multimeric protein or polypeptide able to inhibit interaction of leukocyte Fcγ receptors (FcγR) and immunoglobulin G (IgG), said protein or polypeptide comprising two linked Fc binding regions, at least one of which is derived from an FcγR type receptor.

As used herein, the term "soluble" indicates that the protein or polypeptide is not bound to a cellular membrane, and is, accordingly, characterised by the absence or functional disruption of all or a substantial part of the transmembrane (i.e. lipophilic) domain, so that the soluble receptor is devoid of any membrane anchoring function. The cytoplasmic domains may also be absent.

As used herein, the term "Fc binding region" refers to any part or parts of an Fc receptor that is able to bind with an Fc domain of an immunoglobulin molecule (e.g. an Fc fragment produced by papain hydrolysis of an immunoglobulin molecule) including genetically modified versions thereof, as well as synthetic Fc binding polypeptides.

The at least one Fc binding region derived from an PcγR type receptor may be derived, for example, from an FcγR having low affinity for IgG, that is an affinity for IgG of less than 5 x 10⁷ M⁻¹. Such low affinity receptors include FcγRII type receptors (e.g. FcγRIIa, FcγRIIb and FcγRIIc), FcγRIII type receptors (e.g. FcγRIIIa and FcγRIIIb), truncated forms of FcγRI type receptors (e.g. FcγRIa and FcγRIb) such as truncated polypeptides comprising the first and second of the three ectodomains of an FcγRI receptor (Hulett *et al*., 1991; Hulett *et al.,* 1998), and genetically modified versions of FcγR which normally have high affinity for IgG but by virtue of the modifications (e.g. one or more amino acid substitution(s), deletion(s) and/ or addition(s)) show a reduced affinity for IgG of less than 5x10⁷ M⁻¹).

Preferably, the protein or polypeptide is a homomultimer of an Fc binding region derived from an FcγR receptor such as a low affinity FcγR. A suitable Fc binding region consists of all or an Fc binding part or parts of one or more ectodomains of an FcγR receptor. Persons skilled in the art will be able to readily identify Fc binding ectodomains of FcγR receptors since these domains belong to the IgG domain superfamily (Hulett *et al.,* 1994, Hulett *et al.,* 1995, Hulett *et al.,* 1998, and Tamm et aL, 1996) and are typically characterised by "a tryptophan sandwich" (e.g. residues W90 and W113 of FcγRIIa) and other residues (e.g. in FcγRIIa; residues of the ectodomain 1 and ectodomain 2 linker, and the BC (W113-V119), C'E (F132-P137) and FG (G159-Y160) loops of ectodomain 2 (Hulett *et al.,* 1994))

More preferably, the protein or polypeptide is a homomultimer of an Fc binding region of FcγRIIa. A suitable Fc binding region from FcγRIIa consists of all or an Fc binding part or parts of the ectodomains 1 and 2 of FcγRIIa. The FcγRIIa ectodomains 1 and 2 are found within amino acids 1 to 172 of the FcγRIIa amino acid sequence (Hibbs *et al.,* 1988, and ACCESSION NM_021642). An example of an Fc binding part of the FcγRIIa ectodomains 1 and 2 is a fragment comprising amino acids 90 to 174 of the FcγRIIa amino acid sequence, which includes residues of the ectodomain 1 and ectodomain 2 linker and BC (W113-V119), C'E (F132-P137) and FG (G159-Y160) loops of ectodomain 2. X-ray crystallography studies has revealed that within this fragment, amino acids 113-116,129,131,133,134,155,156 and 158-160 make important contributions to the fragment surface that is able to bind to the Fc domain of IgG (International patent specification no WO 2005/075512).

The protein or polypeptide may also be a heteromultimer of an Fc binding region derived from an FcγRII type receptor and an Fc binding region from another source (e.g. an Fc binding region from another Fc receptor type such as another FcγR type or an Fc binding region from other immunoglobulin receptors such as receptors for IgA and IgE). One especially preferred molecule of this kind is a heterodimer of an Fc binding region derived from an FcγRII type receptor (particularly, FcγRIIa) and an Fc binding region derived from an FcγRIII type receptor.

Fc binding regions considered as having been "derived from" a particular Fc receptor include Fc binding regions having an amino acid sequence which is equivalent to that of an Fc receptor as well as Fc binding regions which include one or more amino acid modification(s) of the sequence of the Fc binding region as found in an Fc receptor. Such amino acid modification(s) may include amino acid substitution(s), deletion(s), addition(s) or a combination of any of those modifications, and may alter the biological activity of the Fc binding region relative to that of an Fc receptor (e.g. the amino acid
modification(s) may enhance selectivity or affinity for immune complexes; such modifications at amino acids 133,134,158-161 are described in International patent specification no WO 96/08512). On the other hand, Fc binding regions derived from a particular Fc receptor may include one or more amino acid modification(s) which do not substantially alter the biological activity of the Fc binding region relative to that of an Fc receptor. Amino acid modification(s) of this kind will typically comprise conservative amino acid substitution(s). Exemplary conservative amino acid substitutions are provided in Table 1 below. Particular conservative amino acid substitutions envisaged are: G, A, V, I, L, M; D, E, N, Q; S, C, T; K, R, H: and P, Nα-alkylamino acids. In general, conservative amino acid substitutions will be selected on the basis that they do not have any substantial effect on (a) the structure of the polypeptide backbone of the Fc binding region at the site of the substitution, (b) the charge or hydrophobicity of the polypeptide at the site of the substitution, and/or (c) the bulk of the amino acid side chain at the site of the substitution. Where an Fc binding region including one or more conservative amino acid substitutions is prepared by synthesis, the Fc binding region may also include an amino acid or amino acids not encoded by the genetic code, such as γ-carboxyglutamic acid and hydroxyproline and D-amino acids.

**Table 1 Exemplary conservative amino acid substitutions**

| | Conservative Substitutions |
|---|---|
| Ala | Val*, Leu, Ile |
| Arg | Lys*, Gln, Asn |
| Asn | Gln*, His, Lys, Arg, Asp |
| Asp | Glu*, asn |
| Cys | Ser |
| Gln | Asn*, His, Lys, |
| Glu | Asp*, γ-carboxyglutamic acid (Gla) |
| Gly | Pro |
| His | Asn, Gln, Lys, Arg* |
| Ile | Leu*, Val, Met, Ala, Phe, norleucine (Nle) |
| Leu | Nle, Ile*, Val, Met, Ala, Phe |
| Lys | Arg*, Gln, Asn, ornithine (Orn) |
| Met | Leu*, Ile, Phe, Nle |
| Phe | Leu*, Val, Ile, Ala |
| Pro | Gly*, hydroxyproline (Hyp),Ser, Thr |
| Ser | Thr |
| Thr | Ser |
| Trp | Tyr |
| Tyr | Trp, Phe*, Thr, Ser |
| Val | Ile, Leu*, Met, Phe, Ala, Nle |

| | |
|---|---|
| *indicates preferred conservative substitutions | |

The Fc binding regions are linked through a peptide bond or via a short linker sequence (e.g. a single amino acid or a short peptide of, for example, 2 to 20 amino acids in length).

Preferably, the protein or polypeptide of the present invention comprises a polypeptide wherein the Fc binding regions are linked in a "head to tail" arrangement. That is, the C-terminal ("tail") of a first Fc binding region will be linked to the N-terminal ("head") of a second Fc binding region. There will be two Fc binding regions linked in a head to tail arrangement. The Fc binding regions will be linked through a peptide bond or via a short linker sequence (e.g. a single amino acid or a short peptide of, for example, 2 to 20 amino acids in length or, more preferably, 2 to 15 amino acids in length, 2 to 10 amino acids in length, 2 to 8 amino acids in length, or, most preferably, 2 to 5 amino acids in length). Suitable short linker sequences may be short random sequences or may comprise short non-Fc binding region fragments of FcγR (e.g. short fragments of 20 or fewer amino acids from the proximal region of the membrane stalk of FcγR). The linker sequence may be a synthetic linker sequence such as, for example, GGGGSGGGGS (SEQ ID NO: 4) which has a low susceptibility to proteolysis. Such a linker sequence may be provided in the form of 2 to 5 tandem "'Gly4Ser" units. Linking the Fc binding regions through a peptide bond or a short linker sequence allows for the production of the polypeptide using recombinant expression systems.

In particularly preferred embodiment of a polypeptide according to the invention, the polypeptide comprises two Pc binding regions from FcγRIIa linked in a head to tail arrangement.

And in a further particularly preferred embodiment of a polypeptide according to the invention, the polypeptide comprises two FcγRIIa extracellular regions each comprising ectodomains 1 and 2, wherein said extracellular regions are linked in a head to tail arrangement through a linker comprising 1 to 20 amino acids.

The polypeptide of the present invention may further comprise a carrier protein or polypeptide (i.e. such that the polypeptide is a "fusion" of the carrier protein or polypeptide and said two or more linked Fc binding regions). The carrier protein may be any suitable carrier protein or polypeptide well known to persons skilled in the art, but preferably, is human serum albumin (HSA) or another carrier protein commonly used to improve bioavailability (i.e. through increasing the serum half life of the polypeptide when administered to a subject). Conveniently, the carrier protein can be fused to the polypeptide by expressing the polypeptide as a fusion protein with the said carrier protein in accordance with any of the methods well known to persons skilled in the art.

The polypeptide of the present invention may further comprise other useful linked molecules, for example, ethylene glycol (i.e. to produce a PEGylated polypeptide) to improve bioavailability, complement regulating molecules such a CD46, CD55 and CD59, cytokines (e.g. to enable delivery of cytokines to sites of inflammation) and cytokine receptors.

Further, as mentioned above, fused polypeptide domains that are capable of binding to one another, can be used to produce a protein or polypeptide according to the invention.

Thus, by using, for example, a polypeptide comprising two Fc binding regions fused to a polypeptide domain capable of binding to another polypeptide domain, which may be the same or different and which is fused to another polypeptide comprising two Fc binding regions a protein can be produced which comprises four Fc binding regions (in other words, a dimeric protein comprising four linked Fc binding regions). Conveniently, this can be achieved through the use of an Fc domain of an immunoglobulin (e.g. an IgG such as IgG₁), since such an Fc domain is capable of dimerising (i.e. with another Fc domain). Preferably, the Fc domain is modified (e.g. by amino acid substitution(s) at residues critical for binding with Fc receptors) to prevent "self-binding" of the Fc domain to linked Fc binding regions, as well as to prevent binding to Fc receptors *in vivo.* In one especially preferred modified Fc domain for use in this manner, the Fc domain is derived from IgG₁ (Wines *et al.,* 2000) and comprises amino acid modification at amino acid 234 and/or 235, namely Leu²³⁴ and/or Leu²³⁵. These leucine residues are within the lower hinge region of IgG₁ where the Fc receptor engages with the Fc domain. One or both of the leucine residues may be substituted or deleted to prevent Fc receptor engagement (i.e. binding); for example, one or both of Leu²³⁴ and Leu²¹⁵ may be substituted with alanine (i.e. L234A and/or L235A) or another suitable amino acid(s) (Wines *et al.,* 2000).

Similarly, by using a polypeptide comprising two or more Fc binding regions linked, for example, in a head to tail arrangement through a peptide bond, short linker sequence or other chemical cross-linking, that is fused to a polypeptide domain capable of binding to another polypeptide domain, which may be the same or different and which is fused to another polypeptide comprising two or more linked Fc binding regions, a protein can be produced which comprises at least four Fc binding region (in other words, a multimeric protein comprising four linked Fc binding regions). In a second aspect, the present invention provides a polynucleotide molecule comprising a nucleotide sequence encoding a soluble multimeric protein or polypeptide according to the first aspect.

The polynucleotide molecule may consist in an expression cassette or expression vector (e.g. a plasmid for introduction into a bacterial host cell, or a viral vector such as a baculovirus vector for transfection of an insect host cell, or a plasmid or viral vector such as a lentivirus for transfection of a mammalian host cell).

Thus, in a third aspect, the present invention provides a recombinant host cell containing a polynucleotide molecule comprising a nucleotide sequence encoding a soluble multimeric protein or polypeptide according to the first aspect.

The recombinant host cell may be selected from bacterial cells such as *E. coli,* yeast cells such as *P. pastoris,* insect cells such as Spodoptera Sf9 cells, mammalian cells such as Chinese hamster ovary (CHO), monkey kidney (COS) cells and human embryonic kidney 293 (HEK 293) cells, and plant cells.

In a fourth aspect, the present invention provides a method for producing a protein or polypeptide, the method comprising the steps of;
(i) providing a recombinant host cell containing a polynucleotide molecule comprising a nucleotide sequence encoding a soluble multimeric protein or polypeptide according to the first aspect,
(ii) culturing said host cell in a suitable culture medium and under conditions suitable for expression of said soluble multimeric protein or polypeptide, and
(iii) isolating said soluble multimeric protein or polypeptide from the culture medium.

The protein or polypeptide may be isolated using any of the methods well known to persons skilled in the art. For example, the protein or polypeptide may be readily isolated using metal affinity chromatography techniques or using immobilised IgG or Heat-aggregated IgG (HAGG) chromatography techniques.

In a fifth aspect, the present invention provides a method of treating a subject for an inflammatory disease, said method comprising administering to said subject a soluble multimeric protein or polypeptide according to the first aspect optionally in combination with a pharmaceutically- or veterinary-acceptable carrier or excipient.

The method is suitable for treatment of inflammatory diseases such as IC-mediated inflammatory diseases including RA, ITP, SLE, glomerulonephritis and heparin-induced thromobocytopoenia thrombosis syndrome (HITTS).

The subject will typically be a human, but the method of the fifth aspect may also be suitable for use with other animal subjects such as livestock (e.g. racing horses) and companion animals.

The term "pharmaceutically- or veterinary-acceptable carrier or excipient" is intended to refer to any pharmaceutically- or veterinary-acceptable solvent, suspending agent or vehicle for delivering the protein or polypeptide of the present invention to the subject.

The protein or polypeptide may be administered to the subject through any of the routes well known to persons skilled in the art, in particular intravenous (iv) administration, intradermal (id) administration and subcutaneous (sc) administration and oral and nasal administration. For subcutaneous administration, the administration may be achieved through injection or by a catheter inserted below the skin. Alternatively, subcutaneous administration may be achieved through sustained release implant compositions or injectable depot-forming compositions.

Typically, the protein or polypeptide will be administered at a dose in the range of 0.5 to 15 mg/kg body weight of the subject per day. Persons skilled in the art will, however, realise that the amount of an "effective dose" (i.e. a dose amount that will be effective in treating an inflammatory disease) will vary according to a number of factors including the age and general health of the subject and the severity of the inflammatory disease to be treated. It is well within the skill of persons skilled in the art to identify or optimise an appropriate effective dose amount for each particular subject.

In further aspects of the present invention, there is provided a composition comprising a soluble multimeric protein or polypeptide according to the first aspect optionally in combination with a pharmaceutically- or veterinary-acceptable carrier or excipient, and the use of a soluble multimeric protein or polypeptide in the manufacture of a medicament for the treatment of an inflammatory disease.

Further, the protein or polypeptide of the first aspect is also useful in applications other than the treatment of a subject for an inflammatory disease. That is, the protein or polypeptide can be used in diagnostic assays for detecting circulating immune complexes (IC) associated with the pathology of autoimmune diseases such as RA and SLE, wherein the protein or polypeptide can be used in a step of "capturing" IC (e.g. by binding the protein or polypeptide to a suitable substrate such as an ELISA plate) in place of the typical precipitation step (with polyethylene glycol) employed in such assays. After capturing IC from a sample (e.g. a serum or synovial fluid sample from a subject) to be assayed, the captured IC can be detected by using the protein or polypeptide in a form whereby it is linked to a molecule which might serve as a marker or reporter (e.g. radio-labelled molecules, chemiluminescent molecules, bioluminescent molecules, fluorescent molecules or enzymes such as horseradish peroxidase which can generate detectable signals). Alternatively, the captured IC could be detected or "probed" using antibodies specific for certain autoantigens (e.g. citrullene in RA, DNA in SLE, La/SS-B in Sjøgren's syndrome, and DNA topoisomerase I in scleroderma) to enable the determination of the level of specific autoantigens in circulating IC, which might allow for the development of assays for autoimmune diseases with improved diagnostic or prognostic results. Moreover, in a similar manner, IC captured by the protein or polypeptide of the present invention bound to a suitable substrate, could be detected or "probed" using antibodies specific for certain antigens of infectious pathogens (e.g. bacteria such as Staphylococcus and Streptococcus, parasites such as P. falciparum (malaria) and viruses such as hepatitis C virus (HCV), Epstein-Barr virus (EBV), human immunodeficiency virus (HIV) and arbovirus causative of Dengue fever), to provide information useful in identifying the causative pathogen of an infection, disease prognosis and/or the management of an infection.

Still further, the protein or polypeptide of the present invention is also useful in various bioassays wherein it can usefully inhibit the release of tumour necrosis factor (TNF) from cells including macrophages, dendritic cells (DC) and neutrophils. Moreover, when linked to a molecule which might serve as a marker or reporter such as those mentioned above, the protein or polypeptide can be used in *in vivo* imaging of sites of inflammation.

Yet further, the protein or polypeptide of the present invention is useful for the removal of circulating IC associated with IC-mediated inflammatory diseases, wherein the protein or polypeptide is bound to a suitable substrate such as an inert bead, fibre or other surface and exposed to a biological fluid (particularly blood) from a subject containing IC complexes such that IC are captured and subsequently removed from the biological fluid. The treated biological fluid, which is substantially depleted of IC, can then be returned to the subject from which it was obtained.

In order that the nature of the present invention may be more clearly understood, preferred forms thereof will now be described with reference to the following non-limiting examples.

### EXAMPLES

### Example 1 Production, purification and characterisation of FcR multimer polypeptides

### Materials and Methods

### Construction of FcγRIIa multimer expression vectors

The Fc binding region comprising the ectodomains 1 and 2 of human FcγRIIa were amplified by using the thermostable polymerase Pwo (Roche), the clone Hu3.0 (Hibbs *et al*, 1988, ACCESSION NM_021642) as cDNA template and the primers oBW10 GTAGCTCCCCCAAAGGCTG (SEQ ID NO:1) and oBW11 CTACCCGGGTGAAGAGCTGCCCATG (SEQ ID NO: 2). The half *SnaB*I (all DNA modifying enzymes were from New England Biolabs) and *Sma*I sites are underlined. The blunt PCR product was ligated using T4 DNA ligase into the vector pPIC9 (Invitrogen, Life Technologies) at the EcoRI site filled in with *Klenow* fragment of DNA polymerase I yielding the vector pBAR14. To produce the vector pBAR28 encoding the tandem ectodomains of FcγRIIa, pBAR14 was digested with SnaBI into which site the *SnaB*I/*Sma*I fragment of pBAR14 was ligated.

A baculovirus vector for expressing FcγRIIa multimerised ectodomains was constructed as follows: The fragment encoding the FcγRIIa leader sequence and ectodomains 1 and 2 were obtained from pVL-1392 (Powell *et al,* 1999, and Maxwell *et al,* 1999) by digest with *EcoR*I and *Xba*I, and then ligated into the *EcoR*I/*Xba*I sites of modified pBACPAK9 (Invitrogen Life Tech) in which the BamHI site in the multiple cloning site had first been eliminated by digest with *BamHI,* filling in using *Klenow* fragment of DNA polymerase and re-ligation. This construct, vector pBAR69, was digested with BamHI to which was ligated the *BamHI* fragment of pBAR28 yielding vectors pBAR71, pBAR72 and pBAR73 encoding rsFcγRIIa dimer, trimer and tetramer respectively. Insert sizes were defined by *EcoR*I/*Xba*I digest and the correct orientation of the multimerising BamHI fragment was screened by *Pvu*II digest using standard protocols.

The mammalian expression vectors encoding FcγRIIa monomer and dimer were produced as follows: The FcγRIIa cDNA clone Hu3.0 (Hibbs *et al,* 1988, and DEFINITION: Homo sapiens Fc fragment of IgG, low affinity IIa, receptor (CD32)(FCGR2A), mRNA, ACCESSION NM_021642) was amplified using accuprime *Pfx* PCR (Invitrogen, Life Technologies) and cloned into the Gateway™ vector pDONR™221 (Invitrogen, Life Technologies) using the BP clonase™ reaction according to the manufacturer's instructions (Invitrogen Life Tech) yielding pNB6. PCR using polymerase accuprime Pfx of pNB6 with the primers oBW11 and oBW302 TCTCATCACCACCATCACCACGTCTAGACCCAGCTTTCTTGTACAAAG (SEQ ID NO: 3), digest with *Sma*I and ligation with T4 ligase yielded pBAR390 encoding the rsFcγRIIa with C-terminal hexahistidine tag. Digestion of pBAR390 with *Bam*HI and ligation of the *BamH*I fragment of pBAR28 yielded vector pBAR397, encoding rsFcγRIIa dimer. *Pvu* II digest was then used to screen for the orientation of the dimerising *Bam*HI fragment and sequencing with ABI BigDye3.1 (Applied Biosytems) confirmed the target sequence. The Gateway LR clonase reaction (Invitrogen, Life Technologies) was then used to transfer the FcγRIIa monomer (pBAR390) or dimer (pBAR397) into Gateway™ reading frame-A cassette (Invitrogen, Life Technologies) adapted expression vector pAPEX3P (Evans et *al,* 1995, and Christiansen et *al,* 1996) to give the expression vectors pBAR426 and pBAR427. Likewise, the Gateway LR clonase reaction was used to transfer the FcγRIIa monomer (pBAR390) or dimer (pBAR397) into Gateway™ reading frame-A cassette (Invitrogen, Life Technologies) adapted expression vector pIRESneo (Clontech). Figure 1 shows the polynucleotide sequence (and translated amino acid sequence) for the "head to tail" dimer construct of FcγRIIa within pBAR397 used to construct the expression vector pBAR427. The two repeats are shown as amino acids 1 to 174 (ie the first Fc binding region) and 184 to 362 (ie the second Fc binding region) and are linked via a short (8 amino acid sequence; residues 175 to 182) fragment of the FcγRIIa membrane proximal stalk plus an additional valine residue (residue 183 shown underlined in Figure 1). Amino acids -31 to -1 of the sequence shown in Figure 1 represents the natural leader sequence of FcγRIIa.

### Production of rsFcγRIIα monomer and dimer polypeptides

Expression of recombinant soluble FcγRIIa (rsFcγRIIa) monomer and dimer polypeptides in HEK 293E cells was performed by transfection with 5 µg of plasmid DNA (pBAR426, pBAR427) in 10cm² wells and Lipofectamine 2000 reagent (Invitrogen, Life Technologies) or Transit reagent (BioRad Laboratories) according to the manufacturer's instructions. After 48 hours, the transfected cells were then selected by incubation in 4µg/ml puromycin. Puromycin selected cells were then grown in 1 % FCS supplemented CD293 media (Invitrogen, Life Technologies) to stationary phase. The recombinant product was subsequently purified by chromatography over immobilised Nickel (Qiagen) or cobalt (Clontech) columns and further purified using Superdex 200 or Superdex G75 (Amersham/Pharmacia) size exclusion chromatography.

### Comparison of affinity measurements of rsFcγRIIa monomer and dimer polypeptides

Using a standard BIAcore assay protocol (Wines *et al,* 2001; Wines *et al,* 2003), affinity measurements for purified rsFcγRIIa monomer and dimer were conducted; the rsFcγRIIa monomer or dimer was injected at varying concentrations over immobilised human IgG monomer (Sandoglobulin, Novartis) or heat-aggregated IgG (HAGG, Wines *et al,* 1988; Wines *et al,* 2003) for 60 minutes, after which time the surface was regenerated (Wines *et al,* 2003). The immobilisation of the human IgG monomer on the biosensor surface causes it to be a multivalent array which mimics an immune complex.

### Comparison of inhibitory activity of rsFcγRIIa monomer and dimer polypeptides

Purified rsFcγRIIa monomer and dimer were incubated with increasing concentrations of a solution of human IgG monomer (Sandoglobulin) and dimer-IgG (Wright *et al,* 1985). The amount of free receptor polypeptide was then measured by injecting over immobilised human IgG monomer in accordance with a standard BIAcore assay protocol.

### Inhibition of immune-complex binding to human cells by rsFcγRIIa monomer and dimer polypeptides

Binding of small immune-complexes (represented by dimer-IgG) to human neutrophils (volunteers V1 and V5) was determined in the absence and presence of purified rsFcγRIIa monomer and dimer polypeptides by flow cytometry analysis (Current Protocols in Immunology, Wiley Interscience).

### Inhibition of TNF secretion from immune-complex stimulated MDMs (monocyte-derived macrophages) by rsFcγRIIa monomer and dimer polypeptides

In a first experiment, peripheral mononuclear cells were extracted from human blood (volunteer V5), positively sorted for CD14 expression using an automacs sorter (Miltenyi Biotec) and allowed to differentiate for 24 hours in the presence of M-CSF to MDMs (monocyte-derived macrophages) prior to stimulation with varying concentrations of small immune-complexes (represented by dimer-IgG), in the absence and presence of rsFcγRIIa dimer (in supernatant at 2.5 µg/ml). TNF secretion from the MDMs was then measured by human TNF ELISA according to manufacturers' protocol (BD Pharmingen). In a second experiment, MDMs were similarly produced *ex vivo* from human blood (this time from volunteer V1) and allowed to differentiate for 24 hours prior to stimulation with varying concentrations of small immune-complexes (ie dimer-IgG), in the absence and presence of rsFcγRIIa dimer (in supernatant at 2.5 µg/ml).

### Inhibition of immune complex activation of platelets by rsFcγRIIa dimer polypeptides

Washed platelets were prepared by low speed centrifugation of whole blood (Thai *et al,* 2003) and stimulated with heat-aggregated IgG (HAGG). Activation of platelets was measured by increased surface expression of P-selectin (CD62P) by flow cytometry (Lau *et al,* 2004).

### Results

### Expression of rsFcγRIIa monomer and multimer polypeptides from insect cells

Western blot analysis of infected cell supernatants demonstrated successful production of dimer and trimer forms of recombinant soluble FcγRIIa (Figure 2). Although some trimer polypeptide was detected this was largely cleaved to the dimeric form and the tetramer was not detected being largely cleaved yielding a dimer form. Since the FcγRIIa dimer was intrinsically the most stable, this was further characterised and developed in a mammalian expression system (ie HEK293E cells).

However, since native FcγRIIa can be shed from leukocyte cell surfaces by proteolysis (Astier *et al,* 1994), one strategy for minimising proteolysis of the trimers, tetramers and larger multimers would be to eliminate or, more preferably, replace the membrane proximal stalk linker sequence linking the FcγRIIa extracellular regions. For example, the proteolytic susceptibility of membrane proximal stalk linker sequence could be reduced by one or more amino acid modifications (e.g. one or more amino acid substitution(s), deletion(s) and/or addition(s)) or by otherwise replacing that linker sequence with a synthetic linker sequence such as, for example, GGGGSGGGGS (SEQ ID NO: 4) which has a low susceptibility to proteolysis.

Another strategy for successfully producing trimers, tetramers and larger multimers, would be to link an expressed dimer polypeptide to one or more monomer or other dimer polypeptide(s) by chemical cross-linking. Multimers of FcγRIIa dimers may also be produced by expressing the dimer polypeptide as a fusion protein with an Fc domain (e.g. an IgG Fc domain) which is of itself dimeric and will thus dimerise any fusion partner.

### Expression of rsFcγRIIa monomer and dimer polypeptides from mammalian cells

Protein yield for purified rsFcγRIIa monomer was 3 mg/l (construct pBAR 426) and for the rsFcγRIIa dimer, to ~0.5 mg/l (construct pBAR 427). Figure 3 shows Coomassie-stained SDS-PAGE (12% acrylamide gel, under non-reducing conditions) of fractions collected from the purification of rsFcγRIIa monomer and dimer. The rsFcγRIIa monomer had the expected size of ~30 kDa (a), while the rsFcγRIIa dimer had the expected size of ~60 kDa (b).

### Comparison of affinity measurements of rsFcγRIIa monomer and dimer polypeptides

The results of the assays are shown in Figures 4 and 5. The assays indicated that rsFcγRIIa monomer has a single-binding site with affinity dissociation constant (K_{D}) of 1.7 µM for human IgG monomer and 1.05 µM for HAGG. In the case of the rsFcγRIIa dimer, the binding data best fitted a two-binding site model with affinity dissociation constants (K_{D}) of 3.2 nM (K_{D1}) and 100 nM (K_{D2}) for immobilised human IgG monomer and 2.73 nM (K_{D1}: approximated 300-fold lesser than the K_{D} of monomeric rsFcγRIIa) and 99 nM (K_{D2}) for HAGG.

### Comparison of inhibitory activity of rsFcγRIIa monomer and dimer polypeptides

The experiments conducted to compare the inhibitory activity of the rsFcγRIIa monomer and dimer polypeptides showed that, in solution, rsFcγRIIa monomer (Figure 6a) does not distinguish between human IgG monomer and small immune-complexes (ie represented by dimer-IgG). In contrast, rsFcγRIIa dimer (Figure 6b) in solution, selectively binds to small immune-complexes (ie dimer-IgG) over human IgG monomer.

### Inhibition of immune-complex binding to human cells by rsFcγRIIa monomer and dimer polypeptides

The results of the inhibition assays are shown in Figure 7a and 7b, and these indicate that rsFcγRIIa dimer (IC₅₀=1.1 µg/ml) is -10-fold more active than rsFcγRIIa monomer (IC₅₀= 10.5 µg/ml) at inhibiting small immune-complexes (ie dimer-IgG) from binding to human neutrophils. Further, the results showed that the inhibition of small immune-complexes (dimer-IgG) from binding to human neutrophils by rsFcγRIIa dimer was reproducible with neutrophils from two different individuals, with an IC₅₀ of 0.9-1.1 µg/ml.

### Inhibition of TNF secretion from immune-complex stimulated MDMs (monocyte-derived macrophages) by rsFcγRIIa monomer and dimer polypeptides

The results are shown in Figures 8a and 8b. The rsFcγRIIa dimer appeared to inhibit immune-complex (ie dimer-IgG) stimulated TNF secretion from 24 hour differentiated human MDMs.

### Inhibition of immune complex activation of platelets by rsFcγRIIa dimer polypeptides

Washed human platelets were incubated with HAGG (10 µg/ml) in the presence and absence of rsFcγRIIa dimer at 30 µg/ml for 30 minutes. As shown in Figure 9, it was found that the activation of platelets was inhibited in the presence of the rsFcγRIIa dimer as evidenced by the lesser expression of P-selectin (CD62P).

### Discussion

Recombinant soluble FcγRIIa in the monomeric (rsFcγRIIa monomer) and dimeric (rsFcγRIIa dimer) form was successfully expressed in HEK 293E cells. BIAcore equilibrium binding assays demonstrated that the rsFcγRIIa dimer has an - 300 fold greater avidity for immobilised IgG (Sandoglobulin) than the monomeric receptor (ie the rsFcγRIIa monomer has a K_{D} ~ 1µM while the rsFcγRIIa dimer has a K_{D} ~ 3nM in the interaction with the immobilised IgG). Competition experiments using BIAcore also demonstrated that the rsFcγRIIa dimer selectively binds small immune complexes, and selective inhibitory activity was confirmed in cell based assays using neutrophils from two donors. The rsFcγRIIa dimer also proved to be about 10 times more potent an inhibitor of small IgG immune complex binding than the rsFcγRIIa monomer, and in a standard platelet assay, the rsFcγRIIa dimer was observed to completely inhibit immune complex activation of platelets (ie rsFcγRIIa dimer is a potent inhibitor of cell activation). It is therefore considered that rsFcγRIIa dimer and other soluble multimeric proteins and polypeptides according to the invention show considerable promise for the treatment of IC-mediated inflammatory disease such as RA and SLE.

### Example 2 Production, purification and characterisation of rsFcγRIIa dimer polypeptide

### Materials and Methods

### Production of rsFcγRIIa dimer polypeptides

The FcγRIIa dimer construct described in Example 1 was cloned into a mammalian expression vector under the control of a modified CMV promoter. Stable CHO-S transfectants were then established as follows: CHO-S cells at 90% confluency were harvested, washed three times, and 2x10⁷ cells in 15 ml medium were dispensed into 10 cm petri dishes. Linearised DNA-lipofectamine 2000 complexes (1:2.5 ratio) were then incubated for 5 minutes at room temperature and added dropwise to the cells. Subsequently, cells were incubated at 37°C for 48 hours, and then plated out in limiting dilution in 96-well plates in CD-CHO medium supplemented with 600 µg/ml hygromycin B, 8 mM L-glutamine, 1x HT supplement and 50 µg/ml dextran sulfate. Cells were screened by standard ELISA to detect soluble FcγRIIa protein, and the highest expressing lines were subcloned again by limiting dilution. One clone (#6) secreted FcγRIIa dimer at approximately 40 mg/L and was cultured in shaker flasks at 30°C for optimal protein expression.

Supernatant containing rsFcγRIIa dimer was concentrated by tangential flow filtration and exchanged into 20 mM sodium phosphate pH 7.4 buffer. The sample was then diluted four-fold in 20 mM sodium phosphate and 0.5 M sodium chloride and purified over a HisTrap FF 2×5ml column (GE Healthcare), eluting in 20 mM sodium phosphate, 0.5 M sodium chloride pH 7.4 and 100 mM imidazole. The eluted material was dialysed and purified by ion exchange chromatography, using a 25 ml Q FF column (GE Healthcare) and eluting in 150 mM sodium chloride. The purified material was then dialysed into phosphate buffered saline.

### Blocking of HAGG binding to FcγRIIb with rsFcγRIIa dimer polypeptides

The ability of purified rsFcγRIIa dimer to block immune complex binding to cell surface FcγRIIb was assessed by a flow cytometric assay. Heat-aggregated IgG (HAGG) was incubated with various concentrations of rsFcγRIIa dimer or rsFcγRIIa monomer (R&D Systems, Cat # 1330-CD/CF) for 1 hour at 4°C. These mixtures were then added to 96-well plates containing 10⁵ IIA1.6 cells transfected with human FcγRIIb (IIA1.6 is a mouse B lymphoma line that lacks endogenous FcγR expression). The plates were incubated for 1 hour at 4°C, washed and then stained with an anti-hIgG-FITC conjugate to detect bound HAGG. After washing, the cells were analysed on a FACS Scan flow cytometer using standard protocols.

### Blocking of HAGG-induced platelet activation with rsFcγRIIa dimer polypeptides

Exposure of platelets to HAGG results in activation via FcγRIIa, leading to upregulation of P-selectin (CD62P). The ability of rsFcγRIIa dimer or rsFcγRIIa monomer to block this activation was assessed by a flow cytometric assay. Heat-aggregated IgG (HAGG) was incubated with various concentrations of rsFcγRIIa dimer or rsFcγRIIa monomer (R&D Systems, Cat # 1330-CD/CF) for 1 hour at 4°C. The mixture was then added to 96-well plates containing 3x10⁷ human platelets, which had been previously washed and resuspended in Tyrodes/Hepes buffer supplemented with 1 mM EDTA. After a 30 minute incubation at room temperature, the cells were washed, fixed, and stained for CD62P and GPIIb (CD41) expression by standard methods and analysed on a FACS Scan flow cytometer.

### rsFcγRIIa dimer polypeptides block immune complex-induced MC/9 activation

MC/9 is an FcγR-positive murine mast cell line that becomes activated and releases TNF-α after exposure to immune complexes. The ability of rsFcγRIIa dimer or rsFcγRIIa monomer to block this activation was assessed using immune complexes consisting of ovalbumin and anti-ovalbumin antibody (OVA ICs) as a stimulus. OVA ICs (10 µg) were incubated with various concentrations of rsFcγRIIa dimer or rsFcγRIIa monomer (R&D Systems, Cat # 1330-CD/CF) for 1 hour at room temperature. The mixture was then added to 96-well plates containing 2×10⁵ MC/9 cells, and incubated overnight at 37°C. Supernatant was collected and the amount of TNF-α measured using a commercial ELISA kit (BD Biosciences).

### Results

### Production of rsFcγRIIa dimer polypeptides

Figure 10 shows the analysis of purified rsFcγRIIa material, including SDS-PAGE (under reducing and non-reducing conditions); Western blotting using an anti-FcγRIIa antibody (R&D systems, catalogue number AF1875) and rabbit anti-goat IgG-peroxidase as the detector antibody; and HPLC. The polypeptide migrates as a single band at the expected molecular weight (~50 kD), reacts with anti-FcγRIIa antibody and is >96% pure as determined by HPLC analysis.

### rsFcγRIIa dimer polypeptides block HAGG binding to FcγRIIb

The results of the HAGG binding assay are shown in Figure 11. Both the rsFcγRIIa dimer and rsFcγRIIa monomer were able to completely block the binding of HAGG to cell surface FcγRIIb. However, the rsFcγRIIa dimer (IC50 = 3.9 ng/ml) was over 500-fold more potent than the monomer protein (IC50 = 2082 ng/ml).

### rsFcγRIIa dimer polypeptides block HAGG-induced platelet activation

The results of the platelet activation assay are shown in Figure 12. The percentage of activated platelets (positive for both CD41 and CD62P) after treatment with HAGG alone was defined as 100%. Both rsFcγRIIa dimer and rsFcγRIIa monomer were able to significantly reduce HAGG-induced CD62P upregulation. Titration revealed that the rsFcγRIIa dimer (IC50 = 3.9 µg/ml) was 5-fold more potent than the rsFcγRIIa monomer (IC50 = 20.9 µg/ml).

### rsFcγRIIa dimer polypeptides block immune complex-induced MC/9 activation

The results of the MC/9activation assay are shown in Figure 13. The amount of TNF-α released after incubation with OVA ICs alone was defined as 100%. Both rsFcγRIIa dimer and rsFcγRIIa monomer were able to completely suppress TNF-α release induced by immune complexes. Titration revealed that the rsFcγRIIa dimer (IC50 = 2.1 µg/ml) was 8-fold more potent than the rsFcγRIIa monomer (IC50 = 17.7 µg/ml).

### Discussion

The rsFcγRIIa dimer was successfully expressed in CHO-S cells. Reducing and non-reducing SDS-PAGE showed that the purified rsFcγRIIa dimer was approximately 50 kDa in size, and Western blotting showed that the dimer was specifically bound by anti-FcγRIIa antibodies. The rsFcγRIIa dimer was determined to be 96% pure by HPLC.

Both the rsFcγRIIa dimer and rsFcγRIIa monomer completely blocked binding of HAGG to cell surface FcγRIIb, with the rsFcγRIIa dimer having an approximately 500-fold increased blocking efficiency than the rsFcγRIIa monomer. Similarly, both the rsFcγRIIa dimer and rsFcγRIIa monomer significantly reduced HAGG-induced platelet activation, with the dimer showing approximately 5-fold higher efficacy than the rsFcγRIIa monomer. Further, both the rsFcγRIIa dimer and rsFcγRIIa monomer suppressed mouse mast cell line (MC/9) activation, as measured by TNF-α release, with the dimer showing 8-fold greater efficacy than the monomer.

### Example 3 Engineering and expression of rsFcγRIIa fusion polypeptides

### Materials and Methods

### Construction of rsFcγRIIa fusion expression vectors

Polynucleotides encoding soluble monomer FcγRIIa or soluble dimer FcγRIIa were independently fused to a polynucleotide encoding IgG₁-FcγR1 (L234A, L235A).

The C-terminal of the soluble monomer FcγRIIa polypeptide was operably fused to a human IgG₁ polypeptide at a position on the N-terminal side of the inter-chain disulphide bond in the lower hinge that covalently joins the two Fc portions. Fusion at this position generates a monomeric FcγRIIa-IgG₁-FcγR1 (L234A, L235A) fusion protein which will dimerise with a second Fc domain due to interactions present between covalently associated Fc domains. The IgG hinge region is known for its flexibility, and fusion of the polypeptide comprising the Fc binding region to the N-terminal side of the inter-chain disulphide bond in the lower hinge allows considerable freedom of movement of the Fc binding region.

Similarly, the C-terminal of the soluble dimer FcγRIIa polypeptide was operably fused to a human IgG₁ polypeptide at a position on the N-terminal side of the inter-chain disulphide bond in the lower hinge that covalently joins the two Fc portions.

Polynucleotides encoding soluble monomer FcγRIIa or soluble dimer FcγRIIa were independently fused to a polynucleotide encoding human serum albumin (HSA) in an equivalent manner to that previously described in International patent specification no WO 96/08512. As disclosed in that specification, the HSA was fused to the N-terminal of the rsFcγRIIa monomer. In a similar manner, the HSA was fused to the N-terminal of the rsFcγRIIa dimer.

Polynucleotides encoding the various fusion polypeptides or proteins were operably inserted into pAPEX 3P-xDEST using standard cloning techniques.

### Production of rsFcγRIIa monomer and rsFcγRIIa dimer fusions

The rsFcγRIIa monomer and dimer fusion expression vectors were transiently transfected into CHOP cells and stably transfected in 293E cells using standard methods. Transiently transfected CHOP cell supernatants were immunoprecipitated using anti FcγRIIa antibody 8.2 (Powell *et al.,* 1999) and immunoprecipitates were subjected to non-reducing SDS-PAGE (12%). Western blot analysis was then performed using standard methods and utilising rabbit anti FcγRIIa antibody (Maxwell *et al.,* 1999) as a primary antibody and anti rabbit Ig-HRP as a secondary antibody.

### HAGG-capture ELISA for detection of rsFcγRIIa fusions in transfected CHOP cell supernatants

HAGG-capture ELISAs were performed to measure the Fc binding activity of the rsFcγRIIa fusions. To examine the binding activity of the rsFcγRIIa monomer fusions, a known FcγRIIa monomer standard (Powell *et al.,* 1999) (starting at 0.75 µg) and the protein from an rsFcγRIIa monomer transfected cell (transfection 426) titrated and compared with the binding of protein from cells transfected with rsFcγRIIa monomer fusion to IgG-Fcγ1 (L234A, L235A) (monomer-Fc) and protein from cells transfected with rsFcγRIIa monomer fusion to HSA (HSA-monomer).

To examine the binding activity of rsFcγRIIa dimer fusions, a known FcγRIIa dimer standard (starting at 0.5 µg/ml) and protein from a cell transfected with rsFcγRIIa dimer (transfection 427) were titrated and compared with the binding of protein from cells transfected with rsFcγRIIa dimer fusion to IgG-Fcγ1 (L234A, L235A) (dimer-Fc) and protein from cells transfected with rsFcγRIIa dimer fusion to HSA (HSA-dimer).

### Capture-Tag ELISA for detection of rsFcγRIIa fusions in transfected CHOP cell supernatants

Using a standard ELISA method, plates were coated with anti FcγRIIa antibody 8.2. The rsFcγRIIa fusions were added to the wells and contacted with the 8.2 antibody. The secondary antibody was anti FcγRIIa antibody 8.7-HRP (Powell *et al.,* 1999; Ierino *et al.,* 1993a), which is specific for a different FcγRIIa epitope than antibody 8.2.

The monomeric rsFcγRIIa samples tested included a known rsFcγRIIa monomer (monomer standard starting at 0.75 µg/ml), the supernatant from rsFcγRIIa monomer transfected cell (transfection 426), the supernatant from cells transfected with rsFcγRIIa monomer fusion to IgG-Fcγ1 (L234A, L235A) (monomer-Fc) and the supernatant from cells transfected with rsFcγIIa monomer fusion to HSA (HSA-monomer).

The dimeric rsFcγRIIa samples tested included a known FcγRIIa dimer (dimer standard starting at 0.5 µg/ml), the supernatant from rsFcγRIIa dimer transfected cell (transfection 427), the supernatant from cells transfected with rsFcγRIIa dimer fusion to IgG-Fcγ1 (L234A, L235A) (monomer-Fc) and the supernatant from cells transfected with rsFcγRIIa dimer fusion to HSA (HSA-monomer).

### Results

### Expression of rsFcγRIIa monomer and dimer fusions

On the basis of the activity of purified rsFcγRIIa monomer and rsFcγRIIa dimer, the rsFcγRIIa monomer-IgG-Fcγ1 (L234A, L235A) fusion was secreted at higher levels (approximately 12 µg/ml in 293E cells) than the rsFcγRIIa dimer-IgG-Fcγ1 fusion (approximately 4 µg/ml in 293E cells).

As shown in Figure 14, Western blot analysis indicated that the fusion proteins were present in the supernatant at the expected molecular weight sizes and that they could be successfully produced as distinct proteins without evidence of degradation products.

### HAGG-capture ELISA for detection of rsFcγRIIa fusions in transfected CHOP cell supernatants

As shown in Figure 15(a), rsFcγRIIa monomer fusion to IgG-Fcγ1 (L234A, L235A) (monomer-Fc) was detectably bound in the assay, while rsFcγRIIa monomer fusion to HSA (HSA-monomer) was observed to bind poorly. This result may be explained by the fact that the rsFcγRIIa monomer fusion to IgG-Fcγ1 (L234A, L235A) will be a dimer (of the Fc binding region) as a consequence of dimerisation between the heavy chains of the fused Fc domains whereas rsFcγRIIa monomer fusion to HSA remains monomeric for the Fc binding region.

As shown in Figure 15(b), purified rsFcγRIIa dimer fusion to IgG-Fcγ1 (L234A, L235A) (dimer-Fc) showed binding activity similar to the dimer standard, and rsFcγRIIa dimer fusion to HSA (HSA-monomer) had detectable, but lower, binding activity. In this case, the rsFcγRIIa dimer fusion to IgG-Fcγ1 (L234A, L235A) was, due to dimerisation between the heavy chains of the fused Fc domains, tetrameric (or "tetravalent") for the Fc binding region, whereas rsFcγRIIa dimer fusion to HSA remains dimeric for the Fc binding region.

### Capture-tag ELISA for detection of rsFcγRIIa fusions in transfected CHOP cell supernatants

As shown in Figure 16 (a), rsFcγRIIa monomer fusion to IgG-Fcγ1 (L234A, L235A) and the rsFcγRIIa monomer fusion to HSA are both captured and detectable in this assay. As shown in Figure 16(b), rsFcγRIIa dimer fusion to IgG-Fcγ1 (L234A, L235A) and the rsFcγRIIa dimer fusion to HSA are also both captured and detectable in this assay. Clearly, both the 8.2 epitope used to capture these receptors and the 8.7 epitope used to detect the captured receptors are intact indicating correct folding of the fusions.

### Discussion

The rsFcγRIIa monomer and rsFcγRIIa dimer fusion constructs were expressed from the vector p-APEX 3P-xDST and expressed transiently in CHOP cells and stably in 293E cells. The expressed fusions presented as distinct proteins on Western blot with no evidence of degradation products.

The rsFcγRIIa dimer fusion to IgG-Fcγ1 (L234A, L235A) may show lower expression levels than its monomeric counterpart. However, the expression level of rsFcγRIIa dimer fusion to HSA was nearly equivalent to the expression level of rsFcγRIIa monomer fusion to HSA, as determined by Western blot (Figure 14), and therefore shows considerable promise as a means for large scale production of rsFcγRIIa dimer. Of interest, the rsFcγRIIa dimer fusions showed higher HAGG and anti-FcγRIIa antibody 8.2 binding activity than monomeric counterparts. As mentioned above, this may be explained by the fact that the rsFcγRIIa dimer fusions were dimeric or tetrameric (in the case of the rsFcγRIIa dimer fusion to IgG-Fcγ1 (L234A, L235A)) for the Fc binding region, and as a consequence, possessed a higher apparent binding affinity (avidity) because of this multi-valency. It is anticipated that tetrameric molecules may bind to immune complexes with such affinity that the binding will be substantially irreversible.

### Example 4 Engineering and expression of heterodimeric Fc receptor polypeptides

### Materials and Methods

### Construction of FcγRIIa-FcγRIII heterodimeric expression vectors

The Fc binding region of FcγRIIa and FcγRIII may be independently PCR amplified from cDNA template using appropriate primers as described in Example 1. The regions amplified would encompass the known characteristic residues and motifs of Fc binding regions such as residues of the ectodomain 1 and ectodomain 2 linker (i.e. the D1/D2 junction), and the BC, C'E and FG loops. The polynucleotide sequences for these Fc binding regions are well known to persons skilled in the art.

Blunt-ended PCR products can be ligated using T4 DNA ligase into the vector pPIC9 (Invitrogen, Life Technologies) at the *Eco*RI site filled in with *Klenow* fragment of DNA polymerase I. An operably fused FcγRIIa-FcγRIII heterodimeric polynucleotide may be created from these amplified products using similar PCR and cloning techniques as those described in Example 1. Insert size and orientation may be confirmed by analytical restriction enzyme digestion or DNA sequencing.

The operably fused FcγRIIa-FcγRIII heterodimeric polynucleotide can be cloned into various expression vectors. For example, the FcγRIIa-FcγRIII heterodimeric polynucleotide may be ligated into the *Eco*RI/*Xba*I sites of modified pBACPAK9 (Invitrogen Life Tech) in which the *Bam*HI site in the multiple cloning site had first been eliminated by digest with *Bam*HI, filling in using Klenow fragment of DNA polymerase and re-ligation. Insert sizes may be defined by *Eco*RI/*Xba*I digest and the correct orientation of the multimerising *Bam*HI fragment can be screened by *Pvu*II digest using standard protocols.

Alternatively, the FcγRIIa-FcγRIII heterodimeric polynucleotide can be cloned into mammalian expression vectors. For example, the Gateway LR clonase reaction (Invitrogen, Life Technologies) may be used to transfer operationally fused multimeric Fc receptor polynucleotide fragments into Gateway™ reading frame-A cassette (Invitrogen, Life Technologies) adapted expression vector pAPEX3P (Evans *et al*, 1995, and Christiansen *et al.,* 1996) to give mammalian expression vectors expressing the fused Fc receptor multimers. Likewise, the Gateway LR clonase reaction can be used to transfer the operationally fused multimeric Fc receptor polynucleotide fragments into Gateway™ reading frame-A cassette (Invitrogen, Life Technologies) adapted expression vector pIRESneo (Clontech).

### Discussion

Multimerisation of Fc binding regions generates molecules having higher avidity interactions with Fc domains. Each monomer in the multimer is able to separately interact with the Fc domain of immunoglobulins to give higher avidities. Multimers containing Fc binding domains derived from different Fc receptors may be generated. For example, multimers could be formed from combinations of the Fc binding regions of FcγRI, FgγRIIa, FcγRIIb, FcγRIIIa, FcγRIIIb, FcαRI and FcεRI. Heterodimers could also be formed from combinations of these Fc binding regions. For example, FcγRIIa-FcγRIII, FcγRIIa-FcγRI, and FcγRI-FcγRIII heterodimers could be formed, as well as heterodimers consisting of other combinations of Fc binding regions.

The Fc binding domains of a number of Fc receptors have been defined by mutagenesis or crystallography (IgG and FcγR: Maxwell *et al.* 1999, Radaev *et al.,* 2001, Sondermann *et al.,* 2000, Hulett *et al*., 1988, Hulett *et al*., 1991, Hulett *et al.*, 1994, Hulett *et al.*, 1995; IgE and FcεRI: Garman *et al.,* 2000; IgA and FcαRI interactions: Wines *et al.,* 2001, Herr *et al.,* 2003). Further, comparisons of similar FcR sequences and comparative analysis of Fc receptor structures have been made (Sondermann *et al.,* 2001). These analyses show that related, clearly defined segments of different Fc receptors are capable of interacting with their ligands. Moreover, crystallographic analysis has demonstrated this clearly for the FcγRIIa and IgG interaction in International patent application no PCT/AU2006/000813 compared to crystallographic analyses of FcγRIII and IgG (Radaev *et al.* 2001, Sonderman *et al.,* 2001).

It is clear that these data together with mutagenesis experiments of other Fc receptors indicate that segments from the connecting region between ectodomain 1 and ectodomain 2 of these related Fc receptors, as well as segments from the BC, C'E and FG loops of the second domains of different receptors, interact with their respective ligands. Incorporation of such Fc binding regions into other polypeptides could confer specificity for that immunoglobulin type on the new polypeptide. To this end, Hulett *et al.,* 1991, Hulett *et al.,* 1995, and Maxwell *et al.,* 1999 have demonstrated that the addition of IgG binding regions into proteins that were otherwise unable to bind IgG acquired specificity for IgG. Similarly, it has been observed that the insertion of a series of IgE binding sequences into proteins unable to bind IgE resulted in protein chimaeras with IgE specificity as previously described in WO 96/08512. It can therefore be predicted that in a similar manner, the inclusion of Fc binding regions that interact with IgG from FcγRI or FcγRIII into a protein could confer IgG binding function to that protein, or similarly, the inclusion of Fc binding regions that interact with IgA from CD89 or FcαRI into a protein could confer IgA binding function to that protein. Such sequences could include the loops of the first extracellular domain of FcαRI of CD89 that are known to interact with IgA, such loops would include the BC, C'E and FG loops of domain 1. Important residues include amino acids 35,52 and 81-86 (Wines *et al.,* 2001, Herr *et al.,* 2003). In this way, receptor proteins or peptides containing segments capable of interacting with different classes of immunoglobulins are possible. Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

### REFERENCES

1. Astier A et al. & D Hanau.1994. Human epidermal Langerhans cells secrete a soluble receptor for IgG (Fc gamma RII/CD32) that inhibits the binding of immune complexes to Fc gamma R+ cells. J Immunol.152(1):201*.*
2. Christiansen D et al. & BE Loveland. 1996. Engineering of recombinant soluble CD46: an inhibitor of complement activation. Immunology. 87:348.
3. Emery P et al. & G Seydoux. 2001. Rheumatology (Oxford) 40:699*.*
4. Evans MJ et al. & SP Squinto.1995. Rapid expression of an anti-human C5 chimeric Fab utilizing a vector that replicates in COS and 293 cells. J Immunol Methods. 1995 184:123*.*
5. Garman SC et al., 2000. Structure of the Fc fragment of human IgE bound to its high-affinity receptor Fc epsilonRI alpha. Nature 406(6793):259-66.
6. Herr AB et al., 2003. Insights into IgA-mediated immune responses from the crystal structures of human FcalphaRI and its complex with IgA1-Fc. Nature 423(6940):614-20.
7. Hibbs ML et al. & PM Hogarth. 1988. Molecular cloning of a human immunoglobulin G Fc receptor. Proc Natl Acad Sci USA 85 (7), 2240.
8. Hulett MD et al. & PM Hogarth.1991. Chimeric Fc receptors identify functional domains of the murine high affinity receptor for IgG. J Immunol. 47(6):1863-8*.*
9. Hulett MD et al. & PM Hogarth 1994. Identification of the IgG binding site of the human low affinity receptor for IgG Fc gamma RII. Enhancement and ablation of binding by site-directed mutagenesis. J Biol Chem. 269(21):15287*.*
10. Hulett MD et al. & PM Hogarth. 1995. Multiple regions of human Fc gamma RII (CD32) contribute to the binding of IgG. J Biol Chem. 270(36):21188.
11. Hulett MD & PM Hogarth.1998. The second and third extracellular domains of FcgammaRI (CD64) confer the unique high affinity binding of IgG2a. Mol Immunol. 35(14-15):989.
12. Ierino FL et al. & PM Hogarth.1993a. Rec. soluble human FcγRII: prodn, characterization, and inhibition of the Arthus reaction. J Exp Med 178:1617*.*
13. Ierino FL et al. & PM Hogarth. 1993b. Mapping epitopes of human Fc gamma RII (CDw32) with monoclonal antibodies and recombinant receptors. J. Immunol. 150:1794-803.
14. Lau LM et al. & DE Jackson. 2004. The tetraspanin superfamily member CD151 regulates outside-in integrin alphaIIbbeta 3 signaling and platelet function. Blood. 104(8):2368*.*
15. Maxwell KF et al. & PM Hogarth. 1999. Crystal structure of the human leukocyte Fc receptor, Fc gammaRIIa. Nat Struct Biol 6:437*.*
16. Nabbe KC et al. & WB van den Berg. 2003. Coordinate expression of activating Fc gamma receptors I and III and inhibiting Fc gamma receptor type II in the determination of joint inflammation and cartilage destruction during immune complex-mediated arthritis. Arthritis Rheum 48:255*.*
17. Pflum LR & ML Graeme. 1979. The Arthus reaction in rats, a possible test for anti-inflammatory and anti-rheumatic drugs. Agents Actions 9:184*.*
18. Powell MS et al. & PM Hogarth. 1999. Biochemical analysis and crystallisation of Fc gamma RIIa, the low affinity receptor for IgG. Immunol Lett. 68(1):17*.*
19. Radaev S et al., 2001. The structure of a human type III Fcgamma receptor in complex with Fc. J. Biol. Chem. 276(19):16469-77.
20. Sondermann P et al., 2000. The 3.2-A crystal structure of the human IgG1 Fc fragment-Fc gammaRIII complex. Nature 406(6793):267-73.
21. Sondermann P et al., 2001. Molecular basis for immune complex recognition: a comparison of Fc-receptor structures. J. Mol. Biol. 309(3):737-49.
22. Takai T. 2002. Roles of Fc receptors in autoimmunity. Nat Rev Immunol. 2(8):580*.*
23. Tamm A et al. & RE Schmidt RE. 1996. The IgG binding site of human FcgammaRIIIB receptor involves CC' and FG loops of the membrane-proximal domain. J Biol Chem. 271(7):3659.
24. Thai LeM et al. & DE Jackson. 2003. Physical proximity and functional interplay of PECAM-1 with the Fc receptor Fc gamma RIIa on the platelet plasma membrane. Brood. 102(10):3637.
25. Wines BD & SB Easterbrook-Smith. 1988. Enhancement of the binding of C1q to immune complexes by polyethylene glycol. Mol Immunol. 25(3):263*.*
26. Wines BD et al. & PM Hogarth. 2000. The IgG Fc contains distinct Fc receptor (FcR) binding sites: the leukocyte receptors Fc gamma RI and Fc gamma RIIa bind to a region in the Fc distinct from that recognized by neonatal FcR and protein A. J. Immunol. 164:5313-8
27. Wines BD et al. & PM Hogarth. 2001. The interaction of Fc alpha RI with IgA and its implications for ligand binding by immunoreceptors of the leukocyte receptor cluster. J Immunol. 166(3):1781*.*
28. Wines BD et al. & PM Hogarth. 2003. Soluble FcgammaRIIa inhibits rheumatoid factor binding to immune complexes. Immunology. 109(2):246.
29. Wright JK et al. & JC Jaton.1980. Preparation and characterization of chemically defined oligomers of rabbit immunoglobulin G molecules for the complement binding studies. Biochem J. 187(3):767.

## Claims

1. A soluble multimeric protein or polypeptide able to inhibit interaction of leukocyte Fcγ receptors (FcγR) and immunoglobulin G (IgG), said protein or polypeptide comprising two linked Fc binding regions at least one of which is derived from an FcγR type receptor, wherein said Fc binding regions are linked through a peptide bond or via a short linker sequence in a head to tail arrangement and wherein said protein or polypeptide comprises just two linked Fc binding regions.

2. The protein or polypeptide of claim 1, wherein said at least one Fc binding region from an FcγR type receptor is derived from an FcγRII type receptor, preferably FcγRIIa.

3. The protein or polypeptide of any one of claims 1 to 2, wherein each of said linked Fc binding regions is derived from an FcγR type receptor, preferably the same FcγRII type receptor.

4. A polypeptide according to any one of claims 1 to 3, wherein said Fc binding regions are linked through a linker comprising 1 to 20 amino acids.

5. The polypeptide of claim 3 or 4, further comprising a carrier protein.

6. The polypeptide of any one of claims 1 to 4, further comprising a polypeptide domain capable of binding to another polypeptide domain.

7. The polypeptide of claim 6, wherein the said polypeptide domain is an Fc domain of an immunoglobulin.

8. The polypeptide of claim 7, wherein said Fc domain is derived from IgG₁ and has been modified by substitution of Leu²³⁴ and/or Leu²³⁵.

9. A polynucleotide molecule comprising a nucleotide sequence encoding the protein or polypeptide of any one of claims 1 to 8.

10. A recombinant host cell comprising the polynucleotide molecule of claim 9.

11. A method of producing a protein or polypeptide, said method comprising the following steps:
(i) providing a recombinant host cell according to claim 10,
(ii) culturing said host cell in a suitable culture medium and under conditions suitable for expression of said soluble multimeric protein or polypeptide, and
(iii) isolating said soluble multimeric protein or polypeptide from the culture medium.

12. A protein or polypeptide of any one of claims 1 to 8 for use in the treatment of an inflammatory disease.

13. The protein or polypeptide for use in the treatment of an inflammatory disease according to claim 12, wherein said inflammatory disease is selected from the group consisting of rheumatoid arthritis (RA), immune thrombocytopenic purpura (ITP), systemic lupus erythematosus (SLE), glomerulonephritis and heparin-induced thrombocytopenia thrombosis syndrome (HITTS).

## Patentansprüche

1. Ein lösliches multimeres Protein oder Polypeptid, welches in der Lage ist, die Interaktion zwischen Leukozyten Fcγ Rezeptoren (FcγR) und Immunglobulin G (IgG) zu inhibieren, wobei besagtes Protein oder Polypeptid zwei verbundene Fc-bindende Regionen umfasst, wovon eine von Rezeptoren des FcγR-Typs stammt, wobei besagte Fc-bindende Regionen durch eine Peptidbindung oder eine kurze Linkersequenz in einer Kopf-an-Schwanz Anordnung verbunden sind und wobei besagtes Protein oder Polypeptid lediglich zwei verbundene Fc-bindende Regionen umfasst.

2. Das Protein oder Polypeptid gemäß Patentanspruch 1, wobei besagte mindestens eine Fc-bindende Region eines Rezeptor des FcγR-Typs von einem Rezeptor des FcγRII-Typs stammt, vorzugsweise FcγRIIa.

3. Das Protein oder Polypeptid von irgendeinem der Patentansprüche 1 bis 2, wobei jede der besagten verbundenen Fc-bindenden Regionen von einem Rezeptor des FcγR-Typs stammt, vorzugsweise demselben Rezeptor des FcγRII-Typs.

4. Ein Polypeptid gemäß irgendeinem der Patentanprüche 1 bis 3, wobei besagte Fc-bindende Regionen über einen Linker verbunden sind, der 1 bis 20 Aminosäuren umfasst.

5. Das Polypeptid gemäß Patentanspruch 3 oder 4 weiter umfassend ein "Carrier"-Protein.

6. Das Polypeptid gemäß irgendeinem der Patentansprüche 1 bis 4, weiter umfassend eine Polypeptiddomäne, die in der Lage ist eine andere Polypeptiddomäne zu binden.

7. Das Polypeptid gemäß Patentanpruch 6, wobei besagte Polypeptiddomäne eine Fc-Domäne eines Immunglobulins ist.

8. Das Polypeptid gemäß Patentanspruch 7, wobei besagte Fc-Domäne von IgG₁ stammt und durch Substitution von Leu²³⁴ und/oder Leu²³⁵modifiziert wurde.

9. Ein Polynukleotidmolekül, umfassend eine Nukleotidsequenz, die das Protein oder Polypeptid gemäß irgendeinen der Patentansprüche 1 bis 8 codiert.

10. Eine rekombinante Wirtszelle, umfassend das Polynukleotidmolekül gemäß Patentanspruch 9.

11. Ein Verfahren zur Herstellung eines Proteins oder Polypeptids, besagtes Verfahren umfassen die folgenden Schritte:
(i) Bereitstellen einer rekombinanten Wirtszelle gemäß Patentanspruch 10,
(ii) Kultivierung besagter Wirtszelle in geeignetem Kulturmedium und unter Bedingungen, die für die Expression von besagtem löslichen multimeren Protein oder Polypeptid geeignet sind, und
(iii) Isolierung besagten löslichen multimeren Proteins oder Polypeptid aus dem Kulturmedium.

12. Ein Protein oder Polypeptid gemäß irgendeinem der Patentansprüche 1 bis 8 für die Verwendung in der Behandlung einer inflammatorischen Krankheit.

13. Das Protein oder Polypeptid zur Verwendung in der Behandlung von inflammatorischer Krankheit gemäß Patentanspruch 12, wobei besagte inflammatorische Krankheit ausgewählt ist aus der Gruppe bestehend aus rheumatoide Arthritis (RA), Immunthrombocytopenie (ITP), Systemischer Lupus Erythematosus (SLE), Glomerulonephritis und Heparin-indizierter Thrombocytopenie Syndrom (HITTS).

## Revendications

1. Protéine ou polypeptide multimère soluble capable d'inhiber l'interaction des récepteurs Fcγ (FcγR) des leucocytes et des immunoglobulines G (IgG), ladite protéine ou ledit polypeptide comprenant deux régions de liaison Fc liées dont au moins l'une est dérivée d'un récepteur de type FcγR, où lesdites régions de liaison Fc sont liées par l'intermédiaire d'une liaison peptidique ou par l'intermédiaire d'une séquence lieur courte dans un arrangement tête à queue et où ladite protéine ou ledit polypeptide comprend juste deux régions de liaison Fc liées.

2. Protéine ou polypeptide selon la revendication 1, où ladite au moins une région de liaison Fc provenant d'un récepteur de type FcγR est dérivée d'un récepteur de type FcγRII, de préférence FcγRIIa.

3. Protéine ou polypeptide selon l'une quelconque des revendications 1 ou 2, où chacune desdites régions de liaison Fc liées est dérivée d'un récepteur de type FcγR, de préférence le même récepteur de type FcγRII.

4. Polypeptide selon l'une quelconque des revendications 1 à 3, où lesdites régions de liaison Fc sont liées par l'intermédiaire d'un lieur comprenant 1 à 20 acides aminés.

5. Polypeptide selon la revendication 3 ou 4, comprenant en outre une protéine de transport.

6. Polypeptide selon l'une quelconque des revendications 1 à 4, comprenant en outre un domaine de polypeptide capable de se lier à un autre domaine de polypeptide.

7. Polypeptide selon la revendication 6, où ledit domaine de polypeptide est un domaine Fc d'une immunoglobuline.

8. Polypeptide selon la revendication 7, où ledit domaine Fc est dérivé d'IgG₁ et a été modifié par substitution de Leu²³⁴ et/ou Leu²³⁵.

9. Molécule de polynucléotide comprenant une séquence nucléotidique codant pour la protéine ou le polypeptide selon l'une quelconque des revendications 1 à 8.

10. Cellule hôte recombinante comprenant la molécule de polynucléotide selon la revendication 9.

11. Procédé de production d'une protéine ou d'un polypeptide, ledit procédé comprenant les étapes suivantes :
(i) la fourniture d'une cellule hôte recombinante selon la revendication 10,
(ii) la culture de ladite cellule hôte dans un milieu de culture approprié et dans des conditions appropriées pour l'expression de ladite protéine ou dudit polypeptide multimère soluble, et
(iii) l'isolement de ladite protéine ou dudit polypeptide multimère soluble à partir du milieu de culture.

12. Protéine ou polypeptide selon l'une quelconque des revendications 1 à 8 pour une utilisation dans le traitement d'une maladie inflammatoire.

13. Protéine ou polypeptide pour une utilisation dans le traitement d'une maladie inflammatoire selon la revendication 12, où ladite maladie inflammatoire est choisie dans le groupe comprenant la polyarthrite rhumatoïde (PR), le purpura thrombopénique auto-immun (PTI), le lupus érythémateux systémique (LES), la glomérulonéphrite et le syndrome de thrombose-thrombopénie induite par l'héparine (TIH).
